# EUROPEAN PATENT APPLICATION

(11) **EP 2 422 814 A1**
(43) Date of publication of application: **29.02.2012**
(21) Application number: 11009290.5
(22) Date of filing: 23.07.2004
(51) Int. Cl.: A61K 45/00, A61K 31/4406, A61K 31/5575, A61K 38/18, A61K 38/27, A61P 19/02, A61P 43/00, A61K 45/06

(54) **Remedy for cartilage-related diseases**

(30) Priority: 25.07.2003 JP 2003280191
(62) Divisional of application: 04748097.5
(71) Applicant: Ono Pharmaceutical Co., Ltd., Osaka-shi, Osaka 541-8526 (JP)
(72) Inventor: Toguchida, Junya, Kyoto-shi Kyoto 604-0042 (JP)
(74) Representative: Grünecker, Kinkeldey, Stockmair & Schwanhäusser

(57) **Abstract**

A remedy for cartilage-related diseases containing as the active ingredient a substance having an agonistic activity to EP2 and/or EP3. A substance having an agonistic activity to EP2 and/or EP3 has effects of promoting chondrogenesis, promoting chondrocyte growth, promoting chondrocyte differentiation, inhibiting cartilage calcification and inhibiting cartilage degradation, or effects of promoting integrin mRNA expression, promoting fibronectin mRNA expression, promoting cyclin D1 mRNA expression and inhibiting osteopontin mRNA expression, and therefore, is useful as a remedy for cartilage-related diseases.

## Description

### TECHNICAL FIELD

The present invention relates to an agent for treating cartilage-related disease and an agent for producing cartilage graft comprising as an active ingredient a substance having a selective EP2 and/or EP3 agonist activity.

### BACKGROUND ART

As the articular cartilage lesions which cause pain, movable region limitation and the like, there are various lesions such as osteoarthritis, rheumatoid arthritis, traumatic or osteonecrosis-accompanied osteochondritis dissecans and the like, and particularly, the number of patients of osteoarthritis has been considerably increased with the advance of aging society. Since the articular cartilage tissue is poor in repairing ability, it is known that even a microscopic lesion is difficult to be treated, gradually progresses and finally results in osteoarthritis. Many of the current therapeutic methods are mainly symptomatic therapies such as soothing of inflammation and pain control by non-steroidal anti-inflammatory drugs. Injection of hyaluronic acid preparations also does not result in the regeneration of cartilage tissue. In recent years, transplantation of self-chondrocytes into damaged part of cartilage has been carried out as a new therapeutic method, but has not been established yet as an actually effective therapeutic method because of the certain reasons such as limitation of the object to partial lesions, future problems of the cartilage-collected parts, necessity of a strictly managed culturing facility for the operation and the like. With the advance of aging society, development of a cartilage disorder treating agent is expected in the near future, which can prevent particularly the progress of osteoarthritis from its initial stage morbid state.

It has been reported so far to have the action that controls the damage of the cartilage by administering prostaglandin E2 (Abbreviate it with PGE2) (JP-A-6-227985, US6,133,230). Therefore, it is expected that a prostaglandin receptor (EP) agonist can become an effective agent for treating cartilage-related diseases. Prostaglandin (PG) E2 has been known as a metabolite in the arachidonate cascade. It has been known that PGE2 possesses cytoprotective activity, uterine contractive activity, a pain-inducing effect, a promoting effect on digestive peristalsis, an awakening effect, a suppressive effect on gastric acid secretion, hypotensive activity and diuretic activity and so on. However, since PGE2 itself has a variety of physical activity, there is a fault that the activities other than the aimed activity become side effects.

The existence of the subtype of the PGE2 receptor with a different role is known. The subtype of EP1, EP2, EP3, and EP4 has been identified so far. (Negishi M., et al., J. Lipid Mediators Cell Signaling, 12, 379-391 (1995)). Therefore, it is expected that an agent for treating cartilage-related diseases with few side effects can be developed by examining the relations to those subtypes and the cartilage, and obtaining the compound that acts only on a specific subtype.

It has been reported that non-selective EP agonists have the effects of inhibiting cartilage damage or of stimulating production of chondrocyte matrix (JP-A-6-227985 and US6,133,230). However, the relation between the specific EP subtype and the effects of stimulating the articular cartilage generation, stimulating chondrocyte growth, inhibiting cartilage degradation, inhibiting cartilage degradation, stimulating chondrocyte differentiation or inhibiting cartilage calcification in cartilage disorder has not been reported.

As the selective EP2 agonist which has been reported, compounds described in EP860430, ONO-8815 (JP-A-11-193268 and Japan Journal of Pharmacology, 1999, 79 (Suppl. I), p.604), compounds described in JP-A-2000-128858, compounds described in WO99/33794, compounds described in EP974580, compounds described in WO95/19964, compounds described in WO98/28264, compounds described in WO99/19300, compounds described in EP0911321, AH-13205 (Cardiovascular Drug Reviews, 1993, 11, 2, p.165-179), CP-533536 (Ref. EP11108426), compounds described in WO98/58911, compounds described in US5,698,598, compounds described in US6,376,533, Butaprostor Rioprostil (Ref. US4,132,738), Misoprostol (Ref. US3,965,143), and AY23626 (Advances in Prostaglandin, Thromboxane, and Leukotriene Research, 1987, 17A, p.467-70) are included.

As the selective EP3 agonist which has been reported, compounds described in WO98/34916, compounds described in JP-A-7-215929, compounds described in JP8-239356, compounds described in WO97/05091, compounds described in WO99/25358, compounds described in JP-A-11-012249, compounds described in JP-A-10-168056, compounds described in JP-A-7-233145, TEI-3356 (Ref. US4,692,464 and Prostaglandins, 1994, vol.48, 5, p.275-83), M&B28767 (Ref. JP-B-51-125255 and FEBS Letter, 1994, vol.338, 2, p.170-174), GR63799X (Ref. JP61-249951 and Advances in Prostaglandin, Thromboxane, and Leukotriene Research, 1991, 21A, p.379-82), SC-46275 (Ref. US4,863,961 and Journal of Pharmacology and Experimental Therapeutics, 1991, vol.259, 3, p.1004-7), Enprostil (Ref. US3.985.791) and Sulprostone (Ref. EP0139608) are included.

However, in these reports relating to EP2 or EP3 agonist, the relations with the mechanism of stimulating the cartilage generation, stimulating chondrocyte growth, inhibiting cartilage degradation, inhibiting cartilage degradation, stimulating chondrocyte differentiation or inhibiting cartilage calcification in cartilage disorder, or the use for cartilage disorder have not been described.

### DISCLOSURE OF THE INVENTION

The problem of this invention is in the offer of an agent for treating cartilage-related diseases comprising EP2 and/or EP3 agonist.

The present inventors have found that the expression of EP2 and EP3 be located at epiphysial cartilage. Moreover, as a result of a large variety of functional analysis in chondrocyte or cartilage, they have found that EP2 and EP3 agonists have an effect of stimulating chondrogenesis. The present inventors found this effect for the first time.

The present invention relates to the followings.
1. An agent for treating cartilage-related disease comprising as an active ingredient a substance having an EP2 and/or EP3 agonist activity.
2. The agent for treating cartilage-related disease according to above-mentioned 1, which is an agent for treating cartilage disorder.
3. The agent for treating cartilage-related disease according to above-mentioned 1, which is an agent for producing a cartilage graft.
4. The agent for treating cartilage-related disease according to above-mentioned 2 or 3, which has one or more effects selected from stimulating chondrogenesis, stimulating chondrocyte growth, stimulating chondrocyte differentiation, inhibiting cartilage calcification and inhibiting cartilage degradation.
5. The agent for treating cartilage-related disease according to above-mentioned 3, which is an agent for chondrocyte culture.
6. The agent for treating cartilage-related disease according to above-mentioned 2 or 3, which has one or more effects selected from stimulating integrin mRNA expression, stimulating fibronectin mRNA expression, stimulating cyclin D1 mRNA expression and inhibiting osteopontin mRNA expression.
7. The agent for treating cartilage-related disease according to above-mentioned 4, wherein the one or more effects selected from stimulating chondrogenesis, stimulating chondrocyte growth, stimulating chondrocyte differentiation, inhibiting cartilage calcification and inhibiting cartilage degradation is/are based on one or more effects selected from stimulating integrin mRNA expression, stimulating fibronectin mRNA expression, stimulating cyclin D1 mRNA expression and inhibiting osteopontin mRNA expression on a chondrocyte or a cartilage tissue.
8. The agent for treating cartilage-related disease according to above-mentioned 7, wherein the effect of stimulating chondrocyte growth is based on stimulating cyclin D1 mRNA expression.
9. The agent for treating cartilage-related disease according to above-mentioned 7, wherein the effect of inhibiting cartilage calcification is based on inhibiting osteopontin mRNA expression.
10. An agent for treating cartilage-related disease comprising a combination of one or more substances selected from transforming growth factor-β, insulin-like growth factor, basic fibroblast growth factor, epidermal growth factor, growth hormone and platelet-derived growth factor, and the substance having an EP2 and/or EP3 agonist activity according to above-mentioned 1.
11. A method for treating cartilage-related disease, which comprises administering a substance having an EP2 and/or EP3 agonist activity.
12. A method for producing a cartilage graft, which comprises adding a substance having an EP2 and/or EP3 agonist activity.
13. Use of a substance having an EP2 and/or EP3 agonist activity, for the preparation of an agent for treating cartilage disorder or for the preparation of an agent for producing a cartilage graft.
14. The agent for treating cartilage-related disease according to above-mentioned 1, wherein the substance having an EP2 agonist activity is one or more compounds selected from a compound described in EP860430, a compound described in WO99/33794 a compound described in EP974580, a compound described in WO2003/74483 a compound described in WO95/19964, a compound described in WO98/28264, a compound described in WO99/19300, a compound described in EP0911321, a compound described in US4,132,738 and a compound described in US3,965,143.
15. The agent for treating cartilage-related disease according to above-mentioned 14, wherein the compound is one or more compounds selected from
   (1) (5Z,9β,11α,13E)-17,17-propano-11,16-dihydroxy-9-chloro-20-norprosta-5,13-dienoic acid,
   (2) (5Z,9β,11α,13E)-17,17-propano-11,16-dihydroxy-9-chloroprosta-5,13,19-trienoic acid,
   (3) trans-2-(4-(1-hydroxyhexyl)phenyl)-5-oxocyclopentaneheptanoic acid,
   (4) 2-[3-(4-tert-butylbenzyl)-N-(pyridin-3-ylsulfonyl)aminomethyl]phenoxy]acetic acid,
   (5) [1R[1α,2β(1E,4R*),3α]]-3-hydroxy-2-[4-hydroxy-4-(1-propylcyclobutyl)-1-butenyl]-5-oxocyclopentane-heptanoic acid methyl ester,
   (6) (2R,3R,4R)-4-hydroxy-2-(7-hydroxyheptyl)-3-[(E)-(4RS)-(4-hydroxy-4-methyl-1-octenyl)]cyclopentanone, and
   (7) (+/-)-15-deoxy-16-α,β-hydroxy-16-methyl PGE1 methylester.
16. The agent for treating cartilage-related disease according to above-mentioned 1, wherein the substance having an EP3 agonist activity is one or more compounds selected from a compound described in WO98/34916, a compound described in JP-A-8-239356, a compound described in US4,692,464, a compound described in JP-A-61-249951, a compound described in US4,863,961and a compound described in US3,985,791.
17. The agent for treating cartilage-related disease according to above-mentioned 16, wherein the compound is one or more compounds selected from
   (1) 11α,15α-dimethoxy-9-oxoprosta-5Z,13E-dienoic acid,
   (2) 2-[5-[2-[N-(diphenylmethyl)carbamoyl]ethyl]naphthalen-1-yloxy]acetic acid,
   (3) (1S,5S,6R,7R)-5-[7-hydroxy-6-[3(S)-hydroxy-3-methyl-1(E)-octenyl]bicyclo[3.3.0]oct-2-ene-3-yl]pentanoic acid,
   (4) (-)-[1(R)-[1α(Z),2β(R*),3α]]-7-[3-hydroxy-2-(2-hydroxy-3-phenxypropoxy)-5-oxocyclopentyl]-4-heptenoic acid 4-(benzoylamino)phenylester,
   (5) methyl-7-(2β-(6-(1-cyclopentyl-yl)-4R-hydroxy-4-methyl-1E,5E-hexadienyl)-3α-hydroxy-5-oxo-1R,1α-cyclopentyl)-4Z-heptenoic acid, and
   (6) 9-oxo-11α,15α-dihydroxy-16-phenoxy-17,18,19,20-tetranorprosta-4,5,13-trans-trienoic acid methyl ester.
18. The agent for treating cartilage-related disease according to claim 1, wherein the compound having an EP3 agonist activity is 16-phenoxy-ω-17,18,19,20-tetranor-PGE₂ methylsulfonamide or a salt thereof.
19. A method for screening the agent for treating cartilage-related disease according to above-mentioned 1.

Cartilage is a connective tissue comprising chondrocytes and a cartilage matrix surrounding the same, and is present, for example, in joint, intervertebral disk of spine, rib cartilage, auricle, auditory meatus, pubic symphysis and epiglottis. The cartilage in the invention includes at least these cartilage tissues. The cartilage tissue comprises chondrocytes and a cartilage matrix produced by the chondrocytes. The chondrocytes described in the present description and the claims include a chondrocyte in a cartilage tissue, a separated chondrocyte, an isolated and purified primary-cultured chondrocyte and a chondrocyte strain. On the other hand, the main components of the cartilage matrix are proteoglycan and collagen (type II, type IX or the like).

Cartilage has an important role in the maintenance of living body functions such as abrasion reduction, elasticity keeping or motor function maintenance of epiphysis region. The cartilage-related diseases of the invention are diseases which accompany cartilage disorders, such as rheumatoid arthritis, osteoporosis, osteoarthritis, osteochondral defect, cartilage damage, articular disk damage, meniscus injury, chondrodysplasia, incomplete repair and healing of bone fracture, refracture, achondroplasia, achondrogenesis, bone deformation or spondylosis deformans, dyschondrogenesis, chondrodystrophia, articular chondrocalcinosis, acute purulent arthritis, tuberculous arthritis, syphilitic arthritis, systemic lupus erythematosus, spondylosis deformans, disk herniation, injury by sports, keypuncher's disease, osteosarcoma, myeloma, osteomalacia, rickets, osteitis fibrosa, renal ostaodystrophy or bone Behcet disease, and which cause functional disorders accompanied by the damage of cartilage tissue of the affected part. It can be expected that the therapeutic agent for cartilage-related diseases of the invention can prevent and/or treat the aforementioned diseases, or improve functional disorders accompanied by the diseases. In addition, it can also be expected to directly prevent and/or treat the cartilage disorders themselves found in the aforementioned diseases.

Some of the agents for treating cartilage disorder of the invention exert their therapeutic effect via an effect of stimulating chondrogenesis. The effect of stimulating chondrogenesis means a stimulation of genesis of a cartilage tissue, particularly the cartilage tissue at the epiphysis region, and is an action which also includes function maintenance of cartilage tissues. Some of the actions are effected via any one of stimulating chondrocyte growth, stimulating chondrocyte differentiation, inhibiting cartilage calcification or inhibiting cartilage degradation, or a multiple combination thereof.

Cartilage tissue comprises chondrocyte and the like parenchymal cells and a cartilage matrix as their intercellular substance. The main components of the cartilage matrix are proteoglycan and collagen (type II, type IX or the like). It is known that proteoglycan is concerned in the swelling property peculiar to the cartilage tissue, and collagen fibers are concerned in the rigidity of cartilage. Aggrecan as a cartilage-specific proteoglycan occupies 90% or more of the proteoglycans in the cartilage matrix, and forms a giant molecule through the bonding of chondroitin sulfate chain, keratan sulfate chain and the like glycosaminoglycan chains to the cartilage core protein. The effect of stimulating chondrogenesis by the agent for stimulating chondrogenesis of the invention means the effect to stimulate differentiation and proliferation of tissue constructing parenchymal cells, particularly chondrocytes, and proper production of cartilage matrix. In addition, the maintenance of the function of cartilage tissue by the agent for stimulating chondrogenesis of the invention means control of appropriate balance of cartilage formation and cartilage calcification or cartilage degradation.

Chondrocytes are derived from undifferentiated interstitial stem cells and classified based on the degree of differentiation into cartilage precursor cells, proliferating chondrocytes, mature chondrocytes and hypertrophic chondrocytes. The effect of stimulating chondrocyte differentiation by the agent for stimulating chondrocyte differentiation of the invention means the action to stimulate differentiation from undifferentiated interstitial stem cells or cartilage precursor cells into proliferating chondrocytes or mature chondrocytes concerned in the formation and function maintenance of cartilage tissues.

In the repairing process of a bone tissue, a cartilage tissue is firstly formed, and subsequently differentiated into osteoblasts and substituted by the bone tissue, thereby completing the bone repair. Such a bone formation via cartilage formation is called cartilaginous ossification and is considered to give a bone repair in which growth and maturation of cartilage derived chondrocytes are normal. As the factors which stimulate growth of chondrocytes, transforming growth factor-β (TGF-β), insulin-like growth factor (IGF-I), basic fibroblast growth factor (bFGF), a combination of epidermal growth factor (EGF) with insulin, growth hormone (GH), platelet-derived growth factor (PDGF) and the like are known. The agent for stimulating chondrocyte growth of the invention shows the effect of stimulating chondrocyte growth solely or when used together with the aforementioned growth accelerating factor.

Calcification means a deposition of lime or other insoluble calcium salts, and in general, it means a process in which calcium carbonate and calcium phosphate generated in the forming process of bones and teeth are deposited, and a tissue or non-cellular matter in the living body is hardened. This process is generally found in a cartilage before the cartilage in which calcium salts are deposited in the matrix is changed to a bone tissue, or sometimes in an aged cartilage. Articular chondrocalcinosis which can be exemplified as a disease of cartilage calcification is a typical disease caused by cartilage calcification abnormality. The agent for inhibiting cartilage calcification of the invention generally has a cartilage calcification inhibitory action to prevent excess calcification by inhibiting a process in which ossification of a cartilage occurs due to abnormal acceleration of calcification, so that it can accelerate function maintenance of cartilage tissues.

The cartilage degradation mainly means degradation of cartilage matrix constituting cartilage tissue. For example, in arthritis patients, degradation and denaturation of collagen and proteoglycan are observed, and a protease which degrades cartilage aggrecan has been identified (Journal of Biological Chemistry, 2000, vol. 275, no. 24, pp. 18566 - 73). Since the agent for inhibiting cartilage degradation of the invention has an effect of inhibiting cartilage degradation, it can control functional reduction due to the reduction of the swelling property, elasticity or rigidity possessed by cartilage tissues, by inhibiting degradation of the cartilage matrix without regard to the mechanism.

The substance having an EP2 and/or EP3 agonist activity used in the present invention includes a substance having an EP2 agonist activity, a substance having an EP3 agonist activity and a substance having an EP2 and/or EP3 agonist activity.

The substance having an EP2 agonist activity includes a substance having an EP2 agonist activity selectively and a substance having an EP2 agonist activity specifically.

Substances having an EP2 agonist activity selectively include substances which may have an EP3 agonist activity which is preferably about 1 /10 or less or about 1 /100 or less, more preferably about 1 /1000 or less of the EP2 agonist activity. On the other hand, substances having an agonist activity to EP2 specifically include substances having prostaglandin receptor agonist activities other than EP2 which are respectively about 1/10 or less or about 1/100 or less, preferably about 1/1000 or less, more preferably about 1/10000 or less of the EP2 agonist activity.

The substance having an EP3 agonist activity includes a substance having an EP3 agonist activity selectively and a substance having an EP3 agonist activity specifically.

Substances having an EP3 agonist activity selectively include substances which may have an EP2 agonist activity which is preferably about 1/10 or less or about 1/100 or less, more preferably about 1/1000 or less of the EP3 agonist activity. On the other hand, substances having an EP3 agonist activity specifically include substances having EP agonist activities other than EP3 which are respectively about 1/10 or less or about 1/100 or less, preferably about 1/1000 or less, more preferably about 1/10000 or less of the EP3 agonist activity.

The substance having an EP2 and EP3 agonist activity means the substance having both agonist activities. The substance includes a substance whose agonist activity to EP2 is stronger than that to EP3, a substance whose agonist activity to EP3 is stronger than that to EP2 or a substance having almost equal agonist activity.

The substance having an EP2 and/or EP3 agonist activity of the present invention may have an agonist activity to EP1, EP4 or prostaglandin receptor. Preferred is the substance having the above-described agonist activities which are respectively 1 /10 or less or 1 /100 or less, preferably 1 /1000 or less of the lower one of EP2 or EP3 agonist activities thereof.

Substances having EP1 and EP4 agonist activities which are respectively 1 /10 or less or 1 /100 or less, preferably 1 /1000 or less of the lower one of EP2 or EP3 agonist activities thereof characteristically act on chondrocytes or cartilage tissue selectively. Therefore, such a substance has the effect of stimulating chondrogenesis, stimulating chondrocyte growth, stimulating chondrocyte differentiation, inhibiting cartilage calcification or inhibiting cartilage degradation, and treating cartilage disorders, which are not observed in non-selective EP agonists.

According to the present invention, unless otherwise indicated and as is apparent for those skilled in the art, symbol 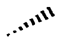 indicates that it is bound to the opposite side of the sheet (namely α-configuration), symbol 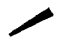 indicates that it is bound to the front side of the sheet (namely β-configuration), symbol 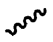 indicates that it is α-configuration, β-configuration or a mixture thereof, symbol 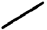 indicates that it is a mixture of α-configuration and β-configuration, 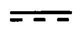 indicates single bond or double bond, 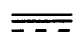 indicates double bond or triple bond, and 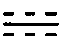 indicates single bond, bond or triple bond.

Unless otherwise specified, isomers are included in the present invention. For example, alkyl, alkenyl, alkynyl, alkoxy, alkylthio, alkylene, alkenylene, alkynylene group includes straight or branched ones. Moreover, isomers on double bond, ring, fused ring (E-, Z-, cis-, trans-isomer), isomers generated from asymmetric carbon atom(s) (R-, S-, α-, β-isomer, enantiomer, diastereomer), optically active isomers (D-, L-, d-, I-isomer), polar compounds generated by chromatographic separation (more polar compound, less polar compound), equilibrium compounds, rotational isomer, mixtures thereof at voluntary ratios and racemic mixtures are also included in the present invention.

A substance having an EP2 agonist activity includes a compound described in EP860430. Moreover, preferred as the compound is compounds represented by formula (1-1) 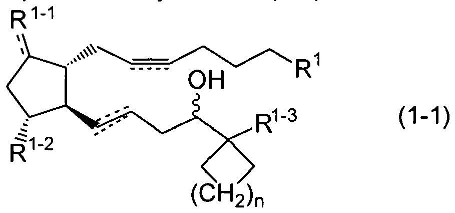 wherein
R¹ is carboxy or hydroxymethyl;
R¹⁻¹ is oxo, methylene or a halogen atom;
R¹⁻² is a hydrogen atom, hydroxy or C1-4 alkoxy;
R¹⁻³ is a hydrogen atom, C1-8 alkyl, C2-8 alkenyl, C2-8 alkynyl, or C1-8 alkyl, C2-8 alkenyl or C2-8 alkynyl substituted by 1 to 3 substituents selected from the following (1) to (5): (1) a halogen atom, (2) C1-4 alkoxy, (3) C3-7 cycloalkyl, (4) phenyl or (5) phenyl substituted by 1 to 3 substituents selected from a halogen atom, C1-4 alkyl, C1-4 alkoxy, nitro or trifluoromethyl; and
n is 0 or 1-4, and
wherein (1) when 5-6 position is triple bond, 13-14 position is not triple bond; and
(2) when 13-14 position is double bond, the double bond represents E form, Z form or mixture of EZ form, or salts thereof.

Among the compound represented by formula (1-1), more preferred is, for example, (5Z,9β,11α,13E)-17,17-propano-11,16-dihydroxy-9-chloro-20-norprosta-5,13-dienoic acid and lysine salt thereof (The compounds are called ONO-8815 and ONO-8815Ly respectively. (Japan Journal of Pharmacology, 1999, 79 (Suppl. I), p.604, JP11-193268).), or (5Z,9β,11α,13E)-17,17-propano-11,16-dihydroxy-9-chloroprosta-5,13,19-trienoic acid (JP-A-2000-128858) or the salt thereof.

In formula (1-1), C1-4 alkyl means, methyl, ethyl, propyl, butyl and the branched isomers thereof, C1-8 alkyl means methyl, ethyl, propyl, butyl, pentyl, hexyl, heptyl, octyl and the branched isomers thereof, C2-8 alkenyl means vinyl, propenyl, butenyl, pentenyl, hexenyl, heptenyl, octenyl and the branched isomers thereof, C2-8 alkynyl means ethynyl, propynyl, butynyl, pentynyl, hexynyl, heptynyl, octynyl and the branched isomers thereof, C1-4 alkoxy means methoxy, ethoxy, propoxy, butoxy and the branched isomers thereof, C3-7 cycloalkyl means cyclopropyl, cyclobutyl, cyclopentyl, cyclohexyl and cycloheptyl, and a halogen atom means fluorine, chlorine, bromine and iodine.

A substance having an EP2 agonist activity includes a compound described in WO99/33794. Moreover, preferred as the compound is compounds represented by formula (1-2) 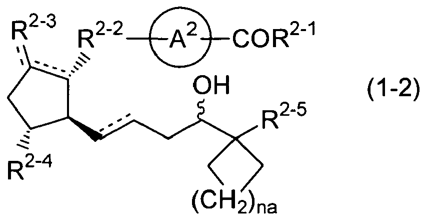 wherein A² is benzene, thiophene or furan ring;
R²⁻¹ is hydroxy, C1-6 alkoxy or NR²⁻¹⁰R²⁻¹¹ group wherein R²⁻¹⁰ and R²⁻¹¹ are independently a hydrogen atom or C1-4 alkyl;
R²⁻² is C1-4 alkylene , C2-4 alkenylene, -S-C1-4 alkylene, -S-C2-4 alkenylene or C1-4 alkylene-S-;
R²⁻³ is oxo, methylene, a halogen atom or R²⁻³²-COO- group wherein R²⁻³² is C1-4 alkyl, C1-4 alkoxy, phenyl, phenyl-C1-4 alkyl, R²⁻³³-OOC-C1-4 alkyl or R²⁻³³-OOC-C2-4 alkenyl in which R²⁻³³ is a hydrogen atom or C1-4 alkyl;
R²⁻⁴ is a hydrogen atom, hydroxy or C1-4 alkoxy;
R²⁻⁵ is C1-8 alkyl, C2-8 alkenyl, C2-8 alkynyl, C1-8 alkyl, C2-8 alkenyl or C2-8 alkynyl substituted by 1 to 3 of substituents selected from the following (1) to (5); (1) a halogen atom, (2) C1-4 alkoxy, (3) C3-7 cycloalkyl, (4) phenyl or (5) phenyl substituted by 1 to 3 substituents selected from a halogen atom, C1-4 alkyl, C1-4 alkoxy, nitro or trifluoromethyl;

na is 0 or an integer of 1-4; and - - - - is a single bond or double bond, and
wherein, when 8-9 position is double bond, R²⁻³ is R²⁻³²-COO- wherein R²⁻³² has the same meaning as described above, and R²⁻¹ is C1-6 alkoxy, or salts thereof.

In formula (1-2), C1-4 alkyl in R²⁻¹¹, R²⁻¹², R²⁻³², R²⁻³³ and R²⁻⁵ means methyl, ethyl, propyl, butyl and the isomers thereof, C1-8 alkyl represented by R²⁻⁵ means methyl, ethyl, propyl, butyl, pentyl, hexyl, heptyl, octyl and the isomers thereof, C1-4 alkoxy represented by R²⁻³², R²⁻⁴ and R²⁻⁵ means methoxy, ethoxy, propoxy, butoxy and the isomers thereof, C1-6 alkoxy represented by R²⁻¹ means methoxy, ethoxy, propoxy, butoxy, pentyloxy, hexyloxy and the isomers thereof, C2-4 alkenyl in R²⁻³² means vinyl, propenyl, butenyl and the isomers thereof, C1-4 alkylene represented by R²⁻² means methylene, dimethylene, trimethylene, tetramethylene and the isomers thereof, and C2-4 alkylene represented by R²⁻² means vinylene, propenylene, butenylene and the isomers thereof. In formula (1-2), C2-8 alkenyl represented by R²⁻³ means vinyl, propenyl, butenyl, pentenyl, hexenyl, heptenyl, octenyl and the isomer thereof, C2-8 alkynyl represented by R²⁻⁵ means ethynyl, propynyl, butynyl, pentynyl, hexynyl, heptynyl, octynyl and the isomers thereof, C3-7 cycloalkyl represented by R²⁻⁵ means cyclopropyl, cyclobutyl, cyclopentyl, cyclohexyl and cycloheptyl, halogen atom in R²⁻³ and R²⁻⁵ means fluorine, chlorine, bromine and iodine.

Moreover, a substance having an EP2 agonist activity includes a compound described in EP974580. Moreover, preferred as the compound is compounds represented by formula (1-3) 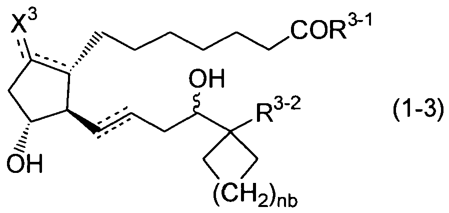 wherein R³⁻¹ is hydroxy, C1-6 alkoxy or NR³⁻¹¹R³⁻¹² group wherein R³⁻¹¹ and R³⁻¹² are independently, a hydrogen atom or C1-6 alkyl;
X³ is a chlorine atom or a fluorine atom;
R³⁻² is a hydrogen atom, C1-8 alkyl, C2-8 alkenyl, C2-8 alkynyl, C1-8 alkyl, C2-8 alkenyl or C2-8 alkynyl substituted by 1 to 3 substituents selected from the following (1) - (5); (1) a halogen atom, (2) C1-4 alkoxy, (3) C3-7 cycloalkyl, (4) phenyl or (5) phenyl substituted by 1 to 3 substituents selected from halogen atom, C1-4 alkyl, C1-4 alkoxy, nitro or trifluoromethyl; and
nb is 0 or an integer of 1-4, or salts thereof.
In formula (1-3), C1-4 alkyl represented by substituents in R³⁻² means methyl, ethyl, propyl, butyl and the isomers thereof, C1-6 alkyl represented by R³⁻¹¹ and R³⁻¹² means methyl, ethyl, propyl, butyl, pentyl, hexyl and the isomers thereof, C1-8 alkyl represented by R³⁻² means methyl, ethyl, propyl, butyl, pentyl, hexyl, heptyl, octyl and the isomers thereof, C2-8 alkenyl represented by R³⁻² means vinyl, propenyl, butenyl, pentenyl, hexenyl, heptenyl, octenyl and the isomers thereof, C2-8 alkynyl represented by R³⁻² means ethynyl, propynyl, butynyl, pentynyl, hexynyl, heptynyl, octynyl and the isomers thereof, C1-4 alkoxy represented by substituents in R³⁻² means methoxy, ethoxy, propoxy, butoxy and the isomers thereof, C1-6 alkoxy represented by R³⁻¹ means methoxy, ethoxy, propoxy, butoxy, pentyloxy, hexyloxy and the isomers thereof, C3-7 cycloalkyl represented by substituents in R³⁻² means cyclopropyl, cyclobutyl, cyclopentyl, cyclohexyl and cycloheptyl, halogen atom represented by substituents in R³⁻² means fluorine, chlorine, bromine and iodine.

Moreover, a substance having an EP2 agonist activity includes a compound described in W02003/74483. Moreover, preferred as the compound is compounds represented by formula (1-4) 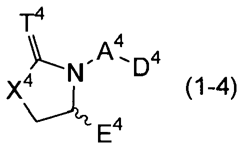 wherein T⁴ is an oxygen atom or a sulfur atom;
X⁴ is -CH₂-, -O- or -S-;
A⁴ is A⁴⁻¹ or A⁴⁻²;
A⁴⁻¹ is C2-8 straight-chain alkylene optionally substituted by 1 to 2 C1-4 alkyl, C2-8 straight-chain alkenylene optionally substituted by 1 to 2 C1-4 alkyl or (3) C2-8 straight-chain alkynylene optionally substituted by 1 to 2 C1-4 alkyl;
A⁴⁻² is -G⁴⁻¹-G⁴⁻²-G⁴⁻³-;
G⁴⁻¹ is C1-4 straight-chain alkylene optionally substituted by 1 to 2 C1-4 alkyl, C2-4 straight-chain alkenylene optionally substituted by 1 to 2 C1-4 alkyl or C2-4 straight-chain alkynylene optionally substituted by 1 to 2 C1-4 alkyl;
G⁴⁻² is -Y⁴-, -ring 1-, -Y⁴-ring 1-, -ring 1-Y⁴⁻ or -Y⁴-C1-4 alkylene-ring 1-;
Y⁴ is -S-, -SO-, -SO₂-, -O- or -NR⁴⁻¹-;
R⁴⁻¹ is a hydrogen atom, C1-10 alkyl or C2-10 acyl,

G⁴⁻³ is a bond, C1-4 straight-chain alkylene optionally substituted by 1 to 2 C1-4 alkyl, C2-4 straight-chain alkenylene optionally substituted by 1 to 2 C1-4 alkyl or C2-4 straight-chain alkynylene optionally substituted by 1 to 2 C1-4 alkyl;
D⁴ is D⁴⁻¹ or D⁴⁻²;
D⁴⁻¹ is -COOH, -COOR⁴⁻², tetrazol-5-yl or -CONR⁴⁻³SO₂R⁴⁻⁴;
R⁴⁻² is C1-10 alkyl, phenyl, C1-10 alkyl substituted by phenyl or biphenyl;
R⁴⁻³ is a hydrogen atom or C1-10 alkyl;
R⁴⁻⁴ is C1-10 alkyl or phenyl;
D⁴⁻² is -CH₂OH, -CH₂OR⁴⁻⁵, hydroxy, -OR⁴⁻⁵, formyl, -CONR⁴⁻⁶R⁴⁻⁷, - CONR⁴⁻⁶SO₂R⁴⁻⁸, -CO-(NH-amino acid residue-CO)ₘ-OH, -O-(CO-amino acid residue-NH)ₘ-H, -COOR⁴⁻⁹, -OCO-R⁴⁻¹⁰, -COO-Z⁴⁻¹-Z⁴⁻²-Z⁴⁻³, 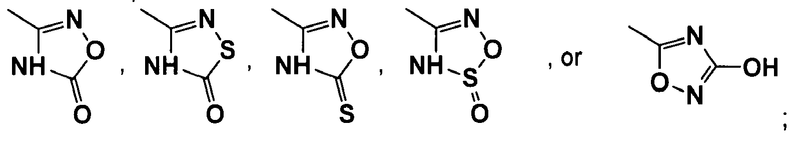 R⁴⁻⁵ is C1-10 alkyl;
R⁴⁻⁶ and R⁴⁻⁷ are, each independently, a hydrogen atom or C1-10 alkyl;
R⁴⁻⁸ is C1-10 alkyl substituted by phenyl;
R⁴⁻⁹ is C1-10 alkyl substituted by biphenyl optionally substituted by 1 to 3 substituents selected from C1-10 alkyl, C1-10 alkoxy and a halogen atom or biphenyl substituted by 1 to 3 substituents selected from C1-10 alkyl, C1-10 alkoxy and a halogen atom;
R⁴⁻¹⁰ is phenyl or C1-10 alkyl;
m is 1 or 2;
Z⁴⁻¹ is C1-15 alkylene, C2-15 alkenylene or C2-15 alkynylene;
Z⁴⁻² is -CO-, -OCO-, -COO-, -CONR^{4-Z1}-, -NR^{4-Z2}CO-, -O-, -S-, -SO₂-, SO₂-NR⁴-, -NR⁴SO₂-, -NR^{4-Z3}-, -NR^{4-Z4}CONR^{4-Z5}-, -NR^{4-Z6}COO-, -OCONR^{4-Z7}- or OCOO-;
Z⁴⁻³ is a hydrogen atom, C1-15 alkyl, C2-15 alkenyl, C2-15 alkynyl, ring Z⁴ or C1-10 alkoxy, C1-10 alkylthio, C1-10 alkyl-NR^{4-Z8}- or C1-10 alkyl substituted by ring Z⁴;
ring Z⁴ is C3-15 mono-, bi- or tri-carbocyclic aryl which may be partially or fully saturated or 3 to 15 membered mono-, bi- or tri-heterocyclic aryl containing 1 to 4 hetero atoms selected from oxygen, nitrogen and sulfur atom which may be partially or fully saturated;

R^{4-Z1}, R^{4-Z2}, R^{4-Z3}, R^{4-Z4}, R^{4-Z5} , R^{4-Z6}, R^{4-Z7} and R^{4-Z8} are, each independently, a hydrogen atom or C1-15 alkyl, wherein R^{4-Z1} and Z⁴⁻³ may be taken together with the nitrogen atom to which they are attached to form 5 to 7 membered saturated mono-heterocyclic ring, the heterocyclic ring may contain other one hetero atom selected from oxygen, nitrogen and sulfur atom, and ring Z⁴ and the saturated mono-heterocyclic ring formed by R^{4-Z1}, Z⁴⁻³ and the nitrogen atom to which they are attached may be substituted by 1 to 3 groups selected from C1-15 alkyl, C2-15 alkenyl, C2-15 alkynyl, C1-10 alkyl substituted by C1-10 alkoxy, C1-10 alkylthio and C1-10 alkyl-NR^{4-Z9}-;
R^{4-Z9} is a hydrogen atom or C1-10 alkyl;
E⁴ is E⁴⁻¹ or E⁴⁻²;
E⁴⁻¹ is 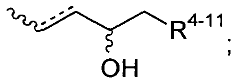 R⁴⁻¹¹ is C1-10 alkyl, C1-10 alkylthio, C1-10 alkyl substituted by ring 2 or C1-10 alkyl substituted by -W⁴⁻¹-W⁴⁻²- ring 2;
W⁴⁻¹ is -O-, -S-, -SO-, -SO₂-, -NR⁴⁻¹¹⁻¹-, carbonyl, -NR⁴⁻¹¹⁻¹SO₂-, carbonylamino or aminocarbonyl;
R⁴⁻¹¹⁻¹ is a hydrogen atom, C1-10 alkyl or C2-10 acyl;
W⁴⁻² is C1-8 alkyl optionally substituted by C1-4 alkyl, a halogen atom or hydroxy;
E⁴⁻² is U⁴⁻¹-U⁴⁻²-U⁴⁻³ or ring 4;
U⁴⁻¹ is C1-4 alkylene, C2-4 alkenylene, C2-4 alkynylene, -ring 3-, C1-4 alkylene-ring 3-, C2-4 alkenylene-ring 3-or C2-4 alkynylene-ring 3-;
U⁴⁻² is a bond, -CH₂-, -CHOH-, -O-, -S-, -SO-, -SO₂-, -NR⁴⁻¹²-, carbonyl, -NR⁴⁻¹²SO₂-, carbonylamino or aminocarbony;
R⁴⁻¹² is a hydrogen atom, C1-10 alkyl or C2-10 acyl;
U⁴⁻³ is C1-8 alkyl optionally substituted by 1 to 3 substituents selected from C1-10 alkyl, halogen, hydroxy, alkoxy, alkylthio and -NR⁴⁻¹³R⁴⁻¹⁴, C1-8 alkenyl optionally substituted by 1 to 3 substituents selected from C1-10 alkyl, a halogen atom, hydroxy, alkoxy, alkylthio and -NR⁴⁻¹³R⁴⁻¹⁴, C1-8 alkynyl optionally substituted by 1 to 3 substituents selected from C1-10 alkyl, a halogen atom, hydroxy, alkoxy, alkylthio and -NR⁴⁻¹³R⁴⁻¹⁴, C1-8 alkyl substituted by ring 4 or ring 4;
R⁴⁻¹³ and R⁴⁻¹⁴ are, each independently, a halogen atom or C1-10 alkyl;
ring 1, ring 2, ring 3 and ring 4 may be substituted by 1 to 5 substituents selected from C1-10 alkyl, C2-10 alkenyl, C2-10 alkynyl, C1-10 alkoxy, C1-10 alkylthio, a halogen atom, hydroxy, nitro, -NR⁴⁻¹⁵R⁴⁻¹⁶, C1-10 alkyl substituted by C1-10 alkoxy, C1-10 alkyl substituted by 1 to 3 halogen atoms, C1-10 alkyl substituted by C1-10 alkoxy substituted by 1 to 3 halogen atoms, C1-10 alkyl substituted by -NR⁴⁻¹⁵R⁴⁻¹⁶, ring 5, -O-ring 5, C1-10 alkyl substituted by ring 5, C2-10 alkenyl substituted by ring 5, C2-10 alkynyl substituted by ring 5, C1-10 alkoxy substituted by ring 5, C1-10 alkyl substituted by -O-ring 5, COOR⁴⁻¹⁷, C1-10alkoxy substituted by 1 to 3 halogen atoms, formyl, C1-10 alkyl substituted by hydroxy or C2-1 0 acyl;
R⁴⁻¹⁵, R⁴⁻¹⁶ and R⁴⁻¹⁷ are, each independently, a hydrogen atom or C1-10 alkyl,
ring 5 may be substituted by 1 to 3 substituents selected from C1-10 alkyl, C2-10 alkenyl, C2-10 alkynyl, C1-10 alkoxy, C1-10 alkyl substituted by C1-10 alkoxy, a halogen atom, hydroxy, C1-10 alkyl substituted by 1 to 3 halogen atoms and C1-10 alkyl substituted by C1-10 alkoxy substituted by 1 to 3 halogen atoms; and
ring 1, ring 2, ring 3, ring 4 and ring 5 are, each independently, C3-15 mono-, bi- or tri-carbocyclic aryl which may be partially or fully saturated or 3 to 15 membered mono-, bi- or tri-heterocyclic aryl containing hetero atoms selected from 1 to 4 nitrogen, 1 to 2 oxygen and/or 1 to 2 sulfur atom which may be partially or fully saturated, and
wherein when E⁴ is E⁴⁻², E⁴⁻² is U⁴⁻¹-U⁴⁻²-U⁴⁻³, and U⁴⁻¹ is C2 alkylene or C2 alkenylene, U⁴⁻² is not -CHOH-;
when U⁴⁻³ is C1-8 alkyl substituted by at least one hydroxy, U⁴⁻¹-U⁴⁻² is not C2 alkylene or C2 alkenylene;
when A⁴ is A⁴⁻¹ and D⁴ is D⁴⁻¹, E⁴ is not E⁴⁻¹;
when T⁴ is an oxygen atom, X⁴ is -CH₂-, D⁴ is D⁴⁻¹, D⁴⁻¹ is COOH, A⁴ is A⁴⁻¹, A⁴⁻¹ is C2-8 straight-chain alkylene, E⁴ is E⁴⁻², E⁴⁻² is U⁴⁻¹-U⁴⁻²-U⁴⁻³, U⁴⁻¹ is C1-4 alkylene and U⁴⁻³ is C1-8 alkyl, U⁴⁻² is not a bond, -CH₂-, -NR¹²- or carbonyl;
when T⁴ is an oxygen atom, X⁴ is -CH₂-, D⁴ is D⁴⁻¹, D⁴⁻¹ is COOH, A⁴ is A⁴⁻², G⁴⁻¹ is C1-4 alkylene, G⁴⁻² is -O- or -NR⁴⁻¹-, G⁴⁻³ is a bond or C1-4 alkylene, E⁴ is E⁴⁻² , E⁴ is U⁴⁻¹-U⁴⁻²-U⁴⁻³, U⁴⁻¹ is C1-4 alkylene and U⁴⁻³ is C1-8 alkyl, U⁴⁻² is not a bond, -CH₂-, -NR⁴⁻¹²- or carbonyl; and
when T⁴ is an oxygen atom, X⁴ is -CH₂-, D⁴ is D⁴⁻¹, E is E⁴⁻², E⁴⁻² is U⁴⁻¹-U⁴⁻²-U⁴⁻³, U⁴⁻¹ is C2 alkylene or C2 alkenylene and U⁴⁻² is -CO-, A⁴ is not A⁴⁻¹, or salts thereof.

In formula (1-4), C1-4 alkyl means methyl, ethyl, propyl, butyl and the isomers thereof, C1-8 alkyl means methyl, ethyl, propyl, butyl, pentyl, hexyl, heptyl, octyl and the isomers thereof, C1-10 alkyl means methyl, ethyl, propyl, butyl, pentyl, hexyl, heptyl, octyl, nonyl, decyl and the isomers thereof, C2-8 alkenyl means ethenyl, propenyl, butenyl, pentenyl, hexenyl, heptenyl, octenyl and the isomers thereof, C2-10 alkenyl means ethenyl, propenyl, butenyl, pentenyl, hexenyl, heptenyl, octenyl, nonenyl, decenyl and the isomers thereof, C2-8 alkynyl means ethynyl, propynyl, butynyl, pentynyl, hexynyl, heptynyl, octynyl and the isomers thereof, C2-10 alkynyl means ethynyl, propynyl, butynyl, pentynyl, hexynyl, heptynyl, octynyl, nonynyl, decynyl and the isomers thereof,C1-4 straight-chain alkylene means methylene, ethylene, trimethylene and tetramethylene, C2-8 straight-chain alkylene means ethylene, trimethylene, tetramethylene, pentamethylene, hexamethylene, heptamethylene and octamethylene, C1-4 alkylene means methylene, ethylene, trimethylene, tetramethylene and the isomers thereof, C2-4 straight-chain alkenylene means ethenylene, propenylene and butenylene.

In formula (1-4), C2-8 straight-chain alkenylene means C2-8 alkenylene which has 1 to 2 double bond(s). It means ethenylene, propenylene, butenylene, butadienylene, pentenylene, pentadienylene, hexenylene, hexadienylene, heptenylene, heptadienylene, octenylene and octadienylene, C2-4 alkenylene means ethenylene, propenylene, butenylene and the isomer thereof, C2-4 straight-chain means alkynylene means ethynylene, propynylene and butynylene, C2-8 alkynylene means C2-8 alkynylene which has 1 to 2 triple bond(s). It means ethynylene, propynylene, butynylene, butadiynylene, pentynylene, pentadiynylene, hexynylene, hexadiynylene, heptynylene, heptadiynylene, octynylene and octadiynylene, C2-4 alkynylene means ethynylene, propynylene, butynylene and the isomers thereof, C1-10 alkoxy means methoxy, ethoxy, propoxy, butoxy, pentyloxy, hexyloxy, heptyloxy, octyloxy, nonyloxy, decyloxy and the isomers thereof.

In formula (1-4), C1-10 alkylthio means methylthio, ethylthio, propylthio, butylthio, pentylthio, hexylthio, heptylthio, octylthio, nonylthio, decylthio and the isomers thereof, C3-8 cycloalkyl means cyclopropyl, cyclobutyl, cyclopentyl, cyclohexyl, cycloheptyl, cyclooctyl, C2-10acyl means ethanoyl, propanoyl, butanoyl , pentanoyl , hexanoyl, heptanoyl, octanoyl, nonanoyl, decanoyl and the isomers thereof, biphenyl means 2-phenylphenyl, 3-phenylphenyl or 4-phenylphenyl, halogen atom means fluorine, chlorine, bromine, iodine.

In formula (1-4), amino acid in -CO-(NH-amino acid residue-CO)ₘ-OH and -O-(CO-amino acid residue-NH)ₘ-H means the amino acid of natural amino acid or abnormal amino acid. Natural amino acids or abnormal amino acid include, for example, glycine, alanine, valine, leucine, isoleucine, serine, threonine, cystein, methionine, proline, asparagine, glutamine, phenylalanine, tyrosine, tryptophan, aspartic acid, glutamic acid, lysine, arginine, histidine, β-alanine, cystathionine, cystine, homoserine, isoleucine, lanthionine, norleucine, norvaline, ornithine, sarcosine, thyronine etc.

In amino acid residue in -CO-(NH-amino acid residue-CO)ₘ-OH and -O-(CO-amino acid residue-NH)ₘ-H, an amino acid with protecting group is included.

In formula (1-4), In the present invention, C3-15 mono-, bi- or tri-carbocyclic aryl which may be partially or fully saturated represented by ring1, ring2 or ring3 includes, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentadecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, teterahydronaphthalene, perhydronaphthalene, heptalene, perhydroheptalene, biphenylene, as-indacene, s-indacene, acenaphthylene, acenaphthene, fluorene, phenalene, phenanthrene, anthracene, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane or noradamantane.

In formula (1-4), among the 3 to 15 membered mono-, bi- or tri-heterocyclic aryl containing hetero atoms selected from 1 to 4 nitrogen, 1 to 2 oxygen, and/or 1 to 2 sulfur atoms which may be partially or fully saturated represented by ring 1, ring 2, ring 3 or ring 4, 3 to 15 membered mono-, bi- or tri-heterocyclic aryl containing hetero atoms selected from 1 to 4 nitrogen, 1 to 2 oxygen, and/or 1 to 2 sulfur atoms includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiin, thianthrene, phenanthridine, phenanthroline, perimidine ring etc.

In formula (1-4), 3 to 15 membered mono-, bi- or tri-heterocyclic aryl containing hetero atoms selected from 1 to 4 nitrogen, 1 to 2 oxygen, and/or 1 to 2 sulfur atoms which is partially or fully saturated includes, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane ring etc.

Moreover, a substance having an EP2 agonist activity includes a compound described in WO95/19964. Moreover, preferred as the compound is compounds represented by formula (1-5-1) 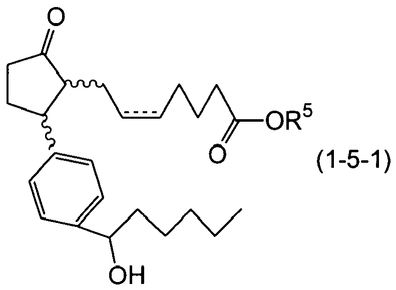 wherein R⁵ is C1-20 saturated or unsaturated non-cyclic hydrocarbon or -(CH₂)ₘₐR⁵⁻¹;
ma is 0 or an integer of 1-10; and
R⁵⁻¹ is C3-7 cycloaliphatic ring or C4-1 0 aryl or heteroaryl ring wherein hetero atom is selected from the group consisting of N, O and S, or salts thereof, and
compounds represented by formula (1-5-2) 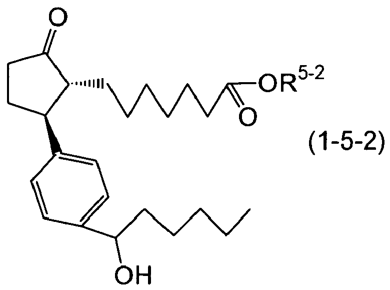 wherein R⁵⁻² is lower alkyl, or salts thereof.

In formulae (1-5-1) and (1-5-2), unless otherwise specified, alkyl means C1-10 alkyl, in which C1-5 lower alkyl is included, cycloalkyl means C3-7 cycloalkyl, and aryl means C4-10 aryl. Saturated or unsaturated non-cyclic hydrocarbon means C1 to about 6 (preferred as 1 to about 4) straight or branched chain hydrocarbon which is saturated or unsaturated. The group includes suitable length alkyl, alkenyl and alkynyl, and preferred is alkyl, for example, methyl, ethyl, propyl, butyl, pentyl, hexyl or isomer thereof. Cycloaliphatic ring is saturated or unsaturated, and preferred is C3-7 saturated ring. As aromatic ring of R⁵⁻¹, preferred is phenyl. Hetero ring has oxygen, nitrogen or sulfur as hetero atom. R⁵⁻¹ may be thienyl, furanyl or pyridyl etc.

In compounds represented by formulae (1-5-1) and (1-5-2), more preferred is, for example, trans-2-(4-(1-hydroxyhexyl)phenyl)-5-oxocyclopentaneheptanoic acid (the compound is called AH-13205 too (Anthony T et. al. and 5 preple. Cardiovascular Drug Reviews, 1993, 11, 2, p.165-179).) or a salt thereof.

Moreover, a substance having an EP2 agonist activity includes a compound described in WO98/28264, WO99/19300 or EP0911321. Preferred as the compound described in WO99/19300 is compounds represented by formula (1-6) 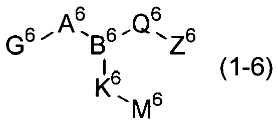 wherein A⁶ is SO₂ or CO;

G⁶ is Ar⁶, Ar⁶⁻¹-V⁶-Ar⁶⁻², Ar⁶-(C1-6) alkylene, Ar⁶-CONH-(C1-6) alkylene, R⁶⁻¹R⁶⁻²-amino, oxy(C1-6) alkylene, amino substituted by Ar⁶ or amino substituted by Ar⁶-(C1-4) alkylene and R⁶⁻¹¹ wherein R⁶⁻¹¹ is a hydrogen atom or C1-8 alkyl,
R⁶⁻¹ and R⁶⁻² may be taken separately and are independently selected from a hydrogen atom and C1-8 alkyl, or R⁶⁻¹ and R⁶⁻² are taken together with the nitrogen atom of the amino group to form a 5 or 6 membered azacycloalkyl, said azacycloalkyl optionally containing an oxygen atom and optionally mono-, di- or tri-substituted independently with up to two oxo, hydroxy, C1-4 alkyl, fluoro or chloro;
B⁶ is a nitrogen atom or CH;
Q⁶ is -(C2-6) alkylene-W⁶-(C1-3) alkylene-, said alkylenes each optionally being substituted with up to four substituents independently selected from a fluorine atom or C1-4 alkyl, -(C4-8) alkylene-, said alkylene being optionally substituted with up to four substituents independently selected from a fluorine atom or C1-4 alkyl, -X⁶-(C1-5) alkylene-, said alkylene being optionally substituted with up to four substituents independently selected from a fluorine atom or C1-4 alkyl, -(C1-5) alkylene-X⁶-, said alkylene being optionally substituted with up to four substituents independently selected from a fluoro atom or C1-4 alkyl, -(C1-3 alkylene)-X⁶-(C1-3) alkylene-, said alkylenes each being optionally substituted with up to four substituents independently selected from a fluorine atom or C1-4 alkyl, -(C1-4) alkylene-W⁶-X⁶-(C0-3) alkylene-, said alkylenes each being optionally substituted with up to four substituents independently selected from a fluorine atom or C1-4 alkyl, -(C0-4 alkylene)-X⁶-W⁶-(C1-3) alkylene-, said alkylenes each being optionally substituted with up to four substituents independently selected from a fluorine atom or C1-4 alkyl, - (C2-5 alkylene)-W⁶-X⁶-W⁶-(C1-3) alkylene-, wherein the two occurrences of W⁶ are independent of each other, said alkylenes each being optionally substituted with up to four substituents each independently selected from a fluorine atom or C1-C4 alkyl, -(C1-4) alkylene-ethenylene-(C1-4) alkylene-, said alkylenes and said ethenylene each being optionally substituted with up to four substituents each independently selected from a fluorine atom or C1-4 alkyl, -(C1-4) alkylene-ethenylene-(C0-2) alkylene-X⁶-(C0-5) alkylene- , said alkylenes and said ethenylene each being optionally substituted with up to four substituents each independently selected from a fluorine atom or C1-4 alkyl, -(C1-4 alkylene)-ethenylene-(C0-2) alkylene-X⁶-W⁶-(C1-3) alkylene- , said alkylenes and said ethenylene each being optionally substituted with up to four substituents each independently selected from a fluorine atom or C1-4 alkyl, - (C1-4) alkylene-ethynylene-(C1-4) alkylene-, said alkylenes and said ethynylene each being optionally substituted with up to four substituents each independently selected from a fluorine atom or C1-4 alkyl, or -(C1-4) alkylene-ethynylene-X⁶-(C0-3) alkylene-, said alkylenes and said ethynylene each being optionally substituted with up to four substituents each independently selected from a fluorine atom or C1-4 alkyl;
Z⁶ is carboxyl, C1-6 alkoxycarbonyl, tetrazolyl, 1,2,4-oxadiazolyl, 5-oxo-1,2,4-oxaziazolyl, 5-oxo-1,2,4-thiadiazolyl, C1-4 alkylsulfonylcarbamoyl, or phenylsulfonylcarbamoyl;
K⁶ is a bond, C1-9 alkylene, thio(C1-4)alkylene, C1-4 alkylenethio(C1-4) alkylene, C1-4 alkyleneoxy(C1-4)alkylene, or oxy(C1-4)alkylene, said C1-9 alkylene being optionally mono-unsaturated and wherein, when K⁶ is not a bond, K⁶ is optionally mono-, di-or tri-substituted independently with a chlorine atom, a fluorine atom, hydroxy or methyl;
M⁶ is -Ar⁶⁻³, -Ar⁶⁻⁴-V1-Ar⁶⁻⁵, -Ar⁶⁻⁴-S-Ar⁶⁻⁵, -Ar⁶⁻⁴-SO-Ar⁶⁻⁵, -Ar⁶⁻⁴-SO₂-Ar⁶⁻⁵, or -Ar⁶⁻⁴-O-Ar⁶⁻⁵,
Ar⁶ is a partially saturated or fully unsaturated 5 to 8 membered ring optionally having 1 to 4 heteroatoms selected independently from an oxygen atom, a sulfur atom and a nitrogen atom, or a bicyclic ring consisting of two fused independently partially saturated, fully saturated or fully unsaturated 5 or 6 membered rings, taken independently, optionally having 1 to 4 heteroatoms selected independently from a nitrogen atom, a sulfur atom and an oxygen atom, or a tricyclic ring consisting of three fused independently partially saturated, fully saturated or fully unsaturated 5 or 6 membered rings, taken independently, optionally having 1 to 4 heteroatoms selected independently from a nitrogen atom, a sulfur atom and an oxygen atom, said partially or fully saturated ring, bicyclic ring or tricyclic ring optionally having 1 or 2 oxo groups substituted on carbon or 1 or 2 oxo groups substituted on sulfur; or Ar⁶ is a fully saturated 5 to 7 membered ring having 1 or 2 heteroatoms selected independently from an oxygen atom, a sulfur atom and a nitrogen atom;
Ar⁶⁻¹ and Ar⁶⁻² are each independently a partially saturated, fully saturated or fully unsaturated 5 to 8 membered ring optionally having 1 to 4 hetero atoms selected independently from an oxygen atom, a sulfur atom and a nitrogen atom, or a bicyclic ring consisting of two fused independently partially saturated, fully saturated or fully unsaturated 5 or 6 membered rings, taken independently, optionally having 1 to 4 heteroatoms selected independently from a nitrogen atom, a sulfur atom and an oxygen atom, or a tricyclic ring consisting of three fused independently partially saturated, fully saturated or fully unsaturated 5 or 6 membered rings, optionally having 1 to 4 hetero atoms selected independently from a nitrogen atom, a sulfur atom and an oxygen atom, said partially or fully saturated ring, bicyclic ring or tricyclic ring optionally having 1 or 2 oxo groups substituted on carbon or 1 or 2 oxo groups substituted on sulfur,
wherein Ar⁶, Ar⁶⁻¹ and Ar⁶⁻² moieties are optionally substituted on carbon or nitrogen, on one ring when the moiety is monocyclic, on one or both rings when the moiety is bicyclic, or on one, two or three rings when the moiety is tricyclic, with up to three substituents per moiety independently selected from R⁶⁻³, R⁶⁻⁴ and R⁶⁻⁵, wherein R⁶⁻³, R⁶⁻⁴ and R⁶⁻⁵ are independently hydroxy, nitro, a halogen atom, carboxy, C1-7 alkoxy, (C1-4)alkoxy(C1-4)alkyl, C1-4 alkoxycarbonyl, C1-7 alkyl, C2-7 alkenyl, C2-7 alkynyl, C3-7 cycloalkyl, (C3-7)cycloalkyl(C1-4)alkyl, (C3-7)cycloalkyl(C1-4)alkanoyl, formyl, C1-8 alkanoyl, (C1-6)alkanoyl(C1-6)alkyl, C1-4 alkanoylamino, C1-4 alkoxycarbonylamino, hydroxysulfonyl, aminocarbonylamino or mono-N-, di-N,N-, di-N,N'- or tri-N,N,N'-(C1-4)alkyl substituted aminocarbonylamino, sulfonamide, C1-4 alkylsulfonamide, amino, mono-N- or di-N,N-(C1-4) alkylamino, carbamoyl, mono-N- or di-N,N-(C1-4 alkyl)carbamoyl, cyano, thiol, C1-6 alkylthio, C1-6 alkylsulfinyl, C1-4 alkylsulfonyl, or mono-N- or di-N,N-(C1-4)alkylaminosulfinyl;
Ar⁶⁻³, Ar⁶⁻⁴ and Ar⁶⁻⁵ are each independently a partially saturated, fully saturated or fully unsaturated 5 to 8 membered ring optionally having 1 to 4 hetero atoms selected independently from an oxygen atom, a sulfur atom and a nitrogen atom, or a bicyclic ring consisting of two fused independently partially saturated, fully saturated or fully unsaturated 5 or 6 membered rings, taken independently, optionally having 1 to 4 heteroatoms selected independently from a nitrogen atom, a sulfur atom and an oxygen atom, or a tricyclic ring consisting of three fused independently partially saturated, fully saturated or fully unsaturated 5 or 6 membered rings, optionally having 1 to 4 hetero atoms selected independently from a nitrogen atom, a sulfur atom and an oxygen atom, said partially or fully saturated ring, bicyclic ring or tricyclic ring optionally having 1 or 2 oxo groups substituted on carbon or 1 or 2 oxo groups substituted on sulfur,
wherein Ar⁶⁻³, Ar⁶⁻⁴ and Ar⁶⁻⁵ moieties are optionally substituted on carbon or nitrogen, on one ring when the moiety is monocyclic, on one or both rings when the moiety is bicyclic, or on one, two or three rings when the moiety is tricyclic, with up to three substituents per moiety independently selected from R⁶⁻³¹ , R⁶⁻⁴¹ and R⁶⁻⁵¹, wherein R⁶⁻³¹ , R⁶⁻⁴¹ and R⁶⁻⁵¹ are independently hydroxy, nitro, a halogen atom, carboxy, C1-7 alkoxy, C1-4 alkoxy(C1-4)alkyl, C1-4 alkoxycarbonyl, C1-7 alkyl, C2-7 alkenyl, C2-7 alkynyl, C3-7 cycloalkyl, (C3-7)cycloalkyl(C1-4)alkyl, (C3-7)cycloalkyl(C1-4)alkanoyl, formyl, C1-8 alkanoyl, (C1-6)alkanoyl(C1-6)alkyl, C1-4 alkanoylamino, C1-4 alkoxycarbonylamino, hydroxysulfonyl, aminocarbonylamino or mono-N-, di-N,N-, di-N,N'- or tri-N,N,N'-(C1-4)alkyl substituted aminocarbonyl, sulfonamide, C1-4 alkylsulfonamide, amino, mono-N- or di-N,N-(C1-4)alkylamino, carbamoyl, mono-N- or di-N,N-(C1-4)alkylcarbamoyl, cyano, thiol, C1-6 alkylthio, C1-6 alkylsulfinyl, C1-4 alkylsulfonyl or mono-N- or di-N,N-(C1-4)alkylaminosulfinyl;
W⁶ is oxy, thio, sulfino, sulfonyl, aminosulfonyl, -mono-N-(C1-4)alkyleneaminosulfonyl, sulfonylamino, N-(C1-4)alkylenesulfonylamino, carboxamide, N-(C1-4)alkylenecarboxamide, carboxamideoxy, N-(C1-4)alkylenecarboxamideoxy, carbamoyl, -mono-N-(C1-4)alkylenecarbamoyl, carbamoyloxy or -mono-N-(C1-4)alkylenecarbamoyloxy, wherein W⁶ alkyl groups are optionally substituted on carbon with 1 to 3 fluorine atoms;
X⁶ is a 5 or 6 membered aromatic ring optionally having 1 or 2 heteroatoms selected independently from an oxygen atom, a nitrogen atom, and a sulfur atom; said ring being optionally mono-, di- or tri-substituted with a halogen atom, (C1-3) alkyl, trifluoromethyl, trifluoromethyloxy, difluoromethyloxy, hydroxyl, (C1-4) alkoxy, or carbamoyl;
R⁶⁻¹, R⁶⁻², R⁶⁻³, R⁶⁻⁴, R⁶⁻⁵, R⁶⁻¹¹, R⁶⁻³¹, R⁶⁻⁴¹ and R⁶⁻⁵¹, when containing an alkyl, alkylene, alkenylene or alkynylene moiety, are optionally mono-, di- or tri-substituted on carbon independently with a halogen atom or hydroxy; and
V and V1 are each independently a bond, thio(C1-4)alkylene, C1-4 alkylenethio, C1-4 alkyleneoxy, oxy(C1-4)alkylene or C1-3 alkylene optionally mono- or di-substituted independently with hydroxy or a fluorine atom, and
wherein (a) when K⁶ is C2-4 alkylene, M⁶ is Ar⁶⁻³ and Ar⁶⁻³ is cyclopent-1-yl, cyclohex-1-yl, cyclohept-1-yl or cyclooct-1-yl, said C5-8 cycloalkyl substituents are not substituted at the one position with hydroxy; and
(b) when K⁶ is a bond; G⁶ is phenyl, phenylmethyl, substituted phenyl or substituted phenylmethyl; Q⁶ is C3-8 alkylene; and M⁶ is Ar⁶⁻³ or Ar⁶⁻⁴-Ar⁶⁻⁵, A is sulfonyl, or salts thereof.

In compounds represented by formula (1-6), more preferred is, for example, 2-[3-(4-tert-butylbenzyl)-N-(pyridin-3-ylsulfonyl)aminomethyl]phenoxy]acetic acid (the compound is called CP-533536 too.) or a salt thereof.

Moreover, a substance having an EP2 agonist activity includes a compound described in WO98/58911. Preferred as the compound is compounds represented by formula (1-7) 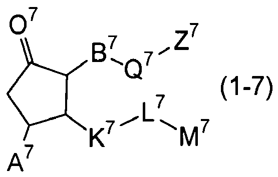 wherein A⁷ is a hydrogen atom or hydroxy;
B⁷ is propylene, propenylene or propynylene;
Q⁷ is propylene, -CH₂OCH₂-, thiazolyl, pyridnyl, phenyl or thienyl;
Z⁷ is carboxyl, C1-6 alkoxycarbonyl, tetrazolyl, 1,2,4-oxadiazolyl or 5-oxo-1,2,4- oxadiazolyl;
K⁷ is ethylene or ethenylene;
L⁷ is a bond or -CO-;
M⁷ is -Ar⁷, -Ar⁷⁻¹-V⁷-Ar⁷⁻², -Ar⁷⁻¹-S-Ar⁷⁻² or -Ar⁷⁻¹-O-Ar⁷⁻²;
Ar⁷ and Ar⁷⁻¹ are either (1) each independently a fully unsaturated 5 to 8 membered ring, which independently and optionally have a bicyclic ring comprising 1 to 4 hetero atoms independently selected from an oxygen atom, a sulfur atom and a nitrogen atom, or two fused partially saturated, fully saturated or fully unsaturated 5 and/or 6 membered rings, independently and optionaly have a tricyclic ring comprising 1 to 4 hetero atoms independently selected from a nitrogen atom, a sulfur atom and an oxygen atom, or three fused partially saturated, fully saturated or fully unsaturated 5 and/or 6 membered rings, and independently and optionally have a partially saturated or fully saturated rings optionally having 1 to 4 hetero atoms selected independently from a nitrogen atom, a sulfur atom and an oxygen atom, or one or more oxo groups substituted on carbon, or
(2) each independently a fully saturated 5 to 8 membered ring;
Ar² is a partially saturated, fully saturated or fully unsaturated 5 to 8 membered ring, wherein Ar² independently and optionally has a bicyclic ring comprising 1 to 4 hetero atoms independently selected from an oxygen atom, a sulfur atom and a nitrogen atom, or two fused partially saturated, fully saturated or fully unsaturated 5 and/or 6 membered rings, independently and optionaly has a tricyclic ring comprising t 1 to 4 hetero atoms independently selected from a nitrogen atom, a sulfur atom and an oxygen atom, or three fused partially saturated, fully saturated or fully unsaturated 5 and/or 6 membered rings, and independently and optionally has a partially saturated or fully saturated rings optionally having 1 to 4 hetero atoms selected independently from a nitrogen atom, a sulfur atom and an oxygen atom, or one or more oxo groups substituted on carbon;
said Ar⁷ and Ar⁷⁻¹ moieties, when a fully unsaturated 5 to 8 membered ring, a bicyclic ring or a tricyclic ring, and said Ar1 moieties are each independently optionally substituted on carbon, on one ring when the moiety is monocyclic, or on two or three rings when the moiety is tricyclic, with up to three substituents selected from R⁷⁻¹, R⁷⁻² and R⁷⁻³ wherein R⁷⁻¹, R⁷⁻² and R⁷⁻³ are independently hydroxy, a nitrogen atom, a halogen atom, C1-7 alkoxy, (C1-4)alkoxy(C1-4)alkyl, C1-4 alkoxycarbonyl, C1-7 alkyl, C2-7 alkenyl, C2-7 alkynyl, C3-7 cycloalkyl, (C3-7)cycloalkyl(C1-4)alkyl, (C3-7)cycloalkyl(C1-4)alkanoyl, formyl, C1-8 alkanoyl, (C1-6)alkanoyl(C1-6)alkyl, aminocarbonylamino, mono-N-, di-N,N-, di-N,N'- or tri-N,N,N-(C1-4)alkyl substituted aminocarbonylamino, C1-4 alkanoylamino, C1-4 alkoxycarbonylamino, sulfonamide, hydrosulfonyl, C1-4 alkylsulfonamide, amino, mono-N-, di-N,N-(C1-4)alkylamino, carbamoyl, mono-N-, di-N,N-(C1-4)alkylcarbamoyl, cyano, thio, C1-6 alkylthio, C1-6 alkylsulfinyl, C1-4 alkylsulfinyl, mono-N-, di-N,N-(C1-4)alkylaminosulfinyl; R⁷⁻¹, R⁷⁻² and R⁷⁻³, when containing an alkyl, alkenyl, alkylene or alkenylene moiety, are optionally straight or branched and are optionally mono-, di- or tri-substituted on carbon independently with halo or hydroxy; and
V is a bond, -CO- or C1-3 alkylene optionally mono- or di-substituted independently with hydroxy or fluoro, wherein (1) when L⁷ is -CO-, A⁷ is hydroxy and (2) when L⁷ is a bond and M⁷ is phenyl, said phenyl is substituted with 1 to 3 substituents selected from R⁷⁻¹, R⁷⁻² and R⁷⁻³, or salts thereof.

Moreover, a substance having an EP2 agonist activity includes a compound described in US5,698,598. Preferred as the compound is compounds represented by formulae (1-8-1), (1-8-2) and (1-8-3) 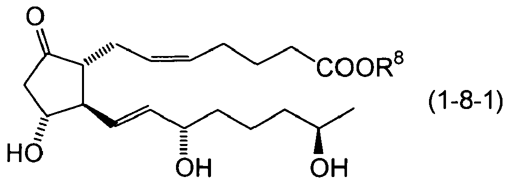 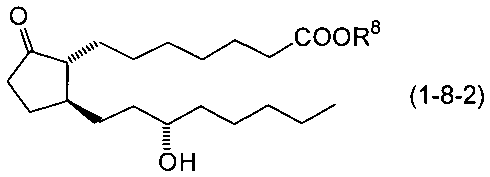 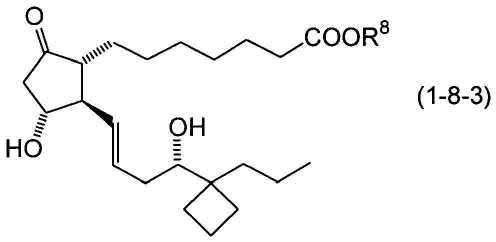 wherein R⁸ is a hydrogen atom, saturated or unsaturated C1-20 cyclic hydrocarbon or -(CH₂)_{mb}R⁸;
mb is 0 or an integer of 1 to 10;
R⁸ is C3-7 aliphatic ring, aryl, C4-10 heteroaryl ring; and
hetero atom is selected from the group consisting of a nitrogen atom, an oxygen atom or a sulfur atom, or salts thereof.

Moreover, a substance having an EP2 agonist activity includes a compound described in US6,376,533. Preferred as the compound is compounds represented by formula (1-9) 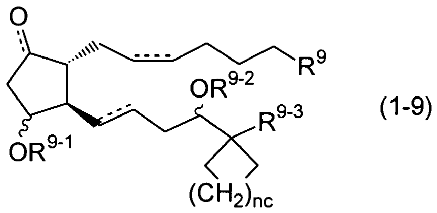 wherein R⁹⁻³ is heteroaryl or optionally substituted heteroaryl;
R⁹⁻¹ and R⁹⁻², each independently, are selected from the group consisting of a hydrogen atom, lower alkyl having up to 6 carbon atoms and lower acyl having up to 6 carbon atoms;
R⁹ is selected from the group consisting of -CO₂R⁹⁻⁴, -CONR⁹⁻⁴,-CH₂OR⁹⁻⁴, -CONR⁹⁻⁴SO₂R⁹⁻⁴, -P(O)(OR⁹⁻⁴) and 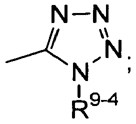 R⁹⁻⁴ is selected from the group consisting of a hydrogen atom, phenyl and C1-6 alkyl; and
nc is 0 or an integer of 1 to 4, or salts thereof.

Moreover, a substance having an EP2 agonist activity includes a compound described in US4,132,738. Preferred as the compound is compounds represented by formula (1-21) 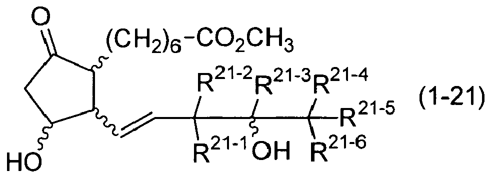 wherein R²¹⁻¹ and R²¹⁻² is a hydrogen atom;
R²¹⁻³ is a hydrogen atom, a C4 methylene chain which is taken together with R²¹⁻⁴ to form a cycloalkyl of up to 6 carbon atoms, or a bicycloalkyl or bicycloalkenyl moiety which is taken together with R²¹⁻⁴ to have the formula 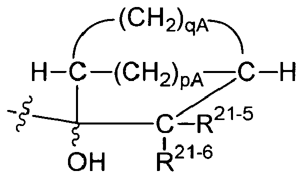 wherein pA is 0 or 1, qA is 2 or 3, and the double bond of such bicycloalkenyl is in the qA bridge;
R²¹⁻⁴ is taken together with R²¹⁻³ to form a cycloalkyl or bicycloalkyl or bicycloalkenyl as defined above, or a methylene chain of 3 carbon atoms which is taken together with R²¹⁻⁵ to form a cycloalkyl of 4 carbon atoms;
R²¹⁻⁵ is a hydrogen atom or taken together with R²¹⁻⁴ to form a cycloalkyl as defined above; and
R²¹⁻⁶ is a hydrogen atom or straight-chain alkyl having 8 carbon atoms, or salts thereof.

In compounds represented by formula (1-21), more preferred is [1 R[1α,2β(1E,4R*),3α]]-3-hydroxy-2-[4-hydroxy-4-(1-propylcyclobutyl)-1-butenyl]-5-oxocyclopentane-heptanoic acid methyl ester (the compound is called Butaprost too.), (2R,3R,4R)-4-hydroxy-2-(7-hydroxyheptyl)-3-[(E)-(4RS)-(4-hydroxy-4-methyl-1-octenyl)]cyclopentanone (the compound is called Rioprostil too.) or salts thereof.

Moreover, a substance having an EP2 agonist activity includes a compound described in US3,965, 143. Preferred as the compound is compounds represented by formula (1-23) 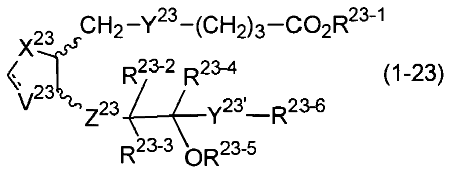 wherein R²³⁻¹, R²³⁻² and R²³⁻³ is a hydrogen atom or C1-7 alkyl;
R²³⁻⁴ is C1-7 alkyl;
R²³⁻⁵ is a hydrogen atom, C1-7 alkyl or C1-7 alkanoyl;
R²³⁻⁶ is C2-4 alkyl or C5-7 cycloalkyl;
X²³ is carbonyl, hydroxymethylene or alkanoyloxymethylene wherein alkanoyl includes 1 to 7 carbon atoms;
V²³ is methylene, hydroxymethylene or alkanoyloxymethylene wherein alkanoyl includes 1 to 7 carbon atoms;
Y²³ is ethylene or vinylene,
Y^{23'} is vinylene, ethynylene or the following group 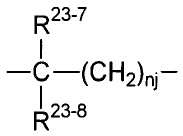 wherein nj is 0 or 1, and Y²³⁻⁷ and Y²³-8 are a hydrogen atom or C1-7 alkyl; and
Z²³ is ethylene, vinylene or ethynylene, or salts thereof.

In compounds represented by formula (1-23), more preferred is, for example, (+/-)-15-deoxy-16-α,β-hydroxy-16-methyl PGE1 methyl ester (the compound is called Misoprostol.) or a salt thereof.

In substances having an EP2 agonist activity of the present invention, preferred is (-)-(S)-15-hydroxy-9-oxo-prostanoic acid (the compound is called AY23626.) or a salt thereof.

On the other hand, a substance having an EP3 agonist activity includes a compound described in WO98/34916. Preferred as the compound is compounds represented by formula (2-10) 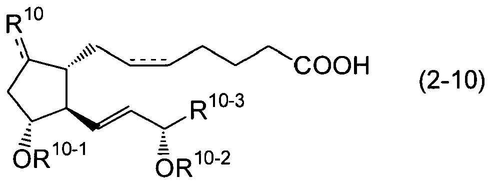 wherein R¹⁰ is oxo or a halogen atom;
R¹⁰⁻¹ and R¹⁰⁻² are each independently C1-4 alkyl, and
R¹⁰⁻³ is C1-10 alkyl, C2-10 alkenylene, C2-10 alkynylene, phenyl, phenoxy, C3-7 cycloalkyl, or C1-10 alkyl, C2-10 alkylene or C2-10 alkenylene substituted by C3-7 cycloalkyloxy, wherein phenyl and cycloalkyl may be substituted by 1 to 3 C1-4 alkyl, C1-4 alkoxy, a halogen atom, trihalomethyl or nitro, or salts thereof.

In compounds represented by formula (2-10), more preferred is 11 α,15α-dimethoxy-9-oxoprosta-5Z,13E-dienoic acid (the compound is called ONO-AE-248 too (ref. WO98/34916).) or a salt thereof.

In formula (2-10), C1-4 alkyl means methyl, ethyl, propyl, butyl and the branched isomers thereof, C1-4 alkoxy means methoxy, ethoxy, propoxy, butoxy and the branched isomers thereof, C1-10 alkyl means methyl, ethyl, propyl, butyl, pentyl, hexyl, heptyl, octyl, nonyl, decyl and the branched isomers thereof, C2-10 alkenyl means vinyl, propenyl, butenyl, pentenyl, hexenyl, heptenyl, octenyl, nonenyl, decenyl and the branched isomers thereof, C2-10 alkynyl means ethynyl, propynyl, butynyl, pentynyl, hexynyl, heptynyl, octynyl, nonynyl, decynyl and the branched isomer thereof, C3-7 cycloalkyl means cyclopropyl, cyclobutyl, cyclopentyl, cyclohexyl, cycloheptyl. In formula (2-10), halogen means fluorine, chlorine, bromine, iodine.

Moreover, a substance having an EP3 agonist activity includes a compound described in JP-A-7-215929. Preferred as the compound is compounds represented by formula (2-11) 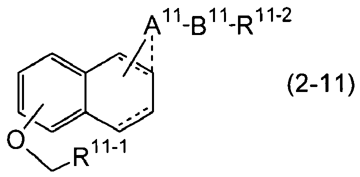 wherein R¹¹⁻¹ is -COOR¹¹⁻⁴ in which R¹¹⁻⁴ is a hydrogen atom or C1-4 alkyl, -CONR¹¹⁻⁵R¹¹⁻⁶ in which R¹¹⁻⁵ and R¹¹⁻⁶ are each independently a hydrogen atom, C1-4 alkyl or C1-4 alkyl substituted with one hydroxy, or - CH₂OH, 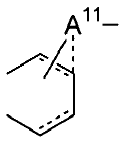 is 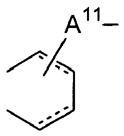 wherein A is a bond or C1-4 alkylene, or 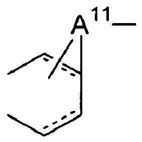 or 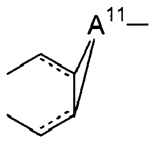 wherein A¹¹ is a group represented by formula 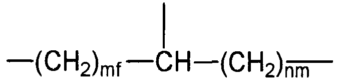 wherein mf and nm are each independently 0 or an integer of 1 to 4 and mf+nm is an integer of 2 to 4;
B¹¹ is -NR¹¹⁻³SO₂- or -SO₂NR¹¹⁻³- wherein R¹¹⁻³ is a hydrogen atom, C1-4 alkyl or -CH₂COOR¹¹⁻⁷ wherein R¹¹⁻⁷ is a hydrogen atom or R^{11-4a} wherein R^{11-4a} is C1-4 alkyl;
R¹¹⁻² is (1) C1-6 alkyl, C2-6 alkenyl or C2-6 alkynyl, (2)C1-6 alkyl, C2-6 alkenyl or C2-6 alkynyl substituted by 1 to 3 substituents selected from phenyl, C4-7 cycloalkyl and phenyl substituted by 1 to 3 substituents selected from C1-4 alkyl, C1-4 alkoxy and a halogen atom or (3) naphthyl; and in 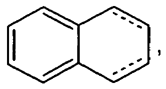 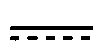 is a bond or double bond, or salts thereof.

Moreover, a substance having an EP3 agonist activity includes a compound described in JP-A-8-239356. Preferred as the compound is compounds represented by formula (2-12) 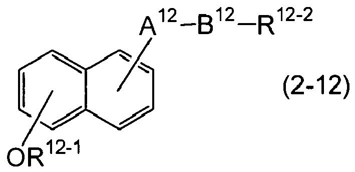 wherein R¹²⁻¹ is a hydrogen atom, C1-4 alkyl, a group represented by formula (C1-4 alkylene)-COOR¹²⁻¹⁰ wherein R¹²⁻¹⁰ is a hydrogen atom or C1-4 alkyl, (C1-4 alkylene)-OH, a group represented by formula (C1-4 alkylene)-CONR¹²⁻⁴R¹²⁻⁵ wherein R¹²⁻⁴ and R¹²⁻⁵ are each independently a hydrogen atom or C1-4 alkyl, a group represented by formula (C1-4 alkylene)-CONR¹²⁻⁶-(C1-4 alkylene)-OH wherein R¹²⁻⁶ is a hydrogen atom or C1-4 alkyl, a group represented by formula (C1-4 alkylene)-NR¹²⁻⁴R¹²⁻⁵ wherein R¹²⁻⁴ and R¹²⁻⁵ have the same meaning as described above, (C1-4 alkylene)-CN or (C1-4 alkylene)-tetrazolyl;
A¹² is a bond, C1-6 alkylene, C2-6 alkenylene, -S-(C1-6 alkylene) or -O-(C1-6 alkylene);
B¹² is a group represented by formula NR¹²⁻³CO or CONR¹²⁻³ wherein R¹²⁻³ is hydrogen or C1-4 alkyl; and
R¹²⁻² is (1) C1-6 alkyl, (2) C2-6 alkenyl, (3) C1-6 alkyl substituted by 1 to 3 substituents optionally selected from phenyl, C4-7 cycloalkyl, naphthyl and 4 to 7 membered heterocycle included one nitrogen, (4) C2-6 alkenyl substituted by 1 to 3 substituents optionally selected from phenyl, C4-7 cycloalkyl, naphthyl and 4 to 7 membered heterocycle included one nitrogen, (5) a group represented by formula R¹²⁻⁷R¹²⁻⁸ wherein R¹²⁻⁷ and R¹²⁻⁸ are each independently phenyl, C4-7 cycloalkyl, naphthyl and 4 to 7 membered heterocycle included one nitrogen or (6) a group represented by formula (C1-6 alkylene)-NR¹²⁻⁷R¹²⁻⁷ wherein R¹²⁻⁷ and R¹²⁻⁸ have the same meanings as described above, and
wherein ring on R¹²⁻² may be substituted by 1 to 3 substituents selected from C1-4 alkyl, C1-4 alkoxy, a halogen atom, nitro and trifluoromethyl, or salts thereof.

In compounds represented by formula (2-12), more preferred is, for example, 2-[5-[2-[N-(diphenylmethyl)carbamoyl]ethyl]naphthalen-1-yloxy]acetic acid (the compound is called ONO-AP-324 too.) or a salt thereof.

Moreover, a substance having an EP3 agonist activity includes a compound described in WO97/05091. Preferred as the compound is compounds represented by formula (2-13) 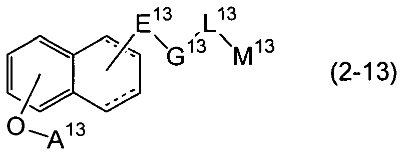 wherein A¹³ is hydrogen, -(C1-4 alkylene)-COOR¹³⁻¹ wherein R¹³⁻¹ is hydrogen or C1-4 alkyl, -(C1-4 alkylene)-CONR¹³⁻²R¹³⁻³ wherein R¹³⁻² and R¹³⁻³ are each independently a hydrogen atom or C1-4 alkyl, -(C1-4 alkylene)-OH, - (C1-4 alkylene)-tetrazolyl or-(C1-4 alkylene)-CN;
E¹³ is a bond or C1-6 alkylene;
G¹³ is -S-, -SO-, -SO₂- -O- or -NR¹³⁻⁴- wherein R¹³⁻⁴ is a hydrogen atom or C1-4 alkyl;
L¹³ is C1-6 alkylene, -(CH₂)_{mc}-CH=CH-(CH₂)_{nd}- wherein mc is 0 or an integer of 1 to 3 and nd is 0 or an integer of 1 to 3, or -(CH₂)ₓₐ-CH(OH)-(CH₂)_{ya}- wherein xa is an integer of 1 to 3 and ya is 0or an integer of 1 to 3;
M¹³ is 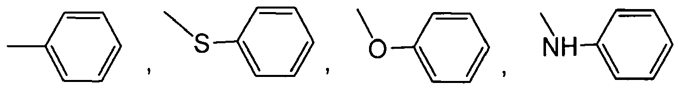 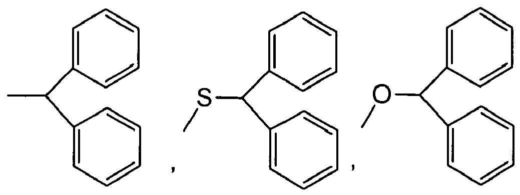 or 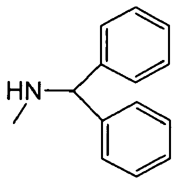 wherein each phenyl in M¹³ may be substituted by 1 to 3 substituents selected from C1-4 alkyl, C1-4 alkoxy, a halogen atom, nitro or trifluoromethyl; and
in 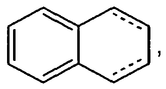 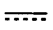 is a bond or double bond, and
wherein (1) when G¹³ is -SO- or -SO₂- M¹³ is not 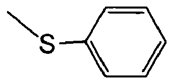 or 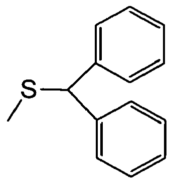 wherein each phenyl in M¹³ may be substituted by 1 to 3 substituents selected from C1-4 alkyl, C1-4 alkoxy, a halogen atom, nitro or trifluoromethyl,
(2) when mc in L¹³ is 0, G¹³ is -SO- or -SO₂-, 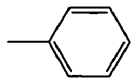 or 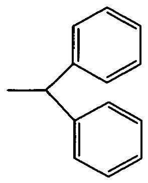
(3) when nd in L¹³ is 0, M¹³ is
   wherein each phenyl in M¹³ may be substituted by 1 to 3 substituents selected from C1-4 alkyl, C1-4 alkoxy, a halogen atom, nitro or trifluoromethyl, 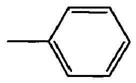 or 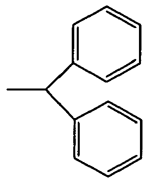
(4) when ya in L¹³ is 0, M¹³ is
   wherein each phenyl in M¹³ may be substituted by 1 to 3 substituents selected from C1-4 alkyl, C1-4 alkoxy, a halogen atom, nitro or trifluoromethyl,
(5) when A¹³ is hydrogen, L¹³ is -(CH₂)_{mc}-CH=CH-(CH₂)_{nd}- wherein mc and nd are the same meanings as described above or -(CH₂)ₓₐ-CH(OH)-(CH₂)_{ya}- wherein xa and ya are the same meanings as described above, and
(6) tetrazolyl in A¹³ is 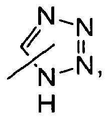 or pharmaceutically acceptable salts
   thereof.

In formula (2-13), C1-4 alkyl represented by R¹³⁻¹, R¹³⁻², R¹³⁻³ and R¹³⁻⁴ or C1-4 alkyl as the substituent of phenyl in M¹³ means methyl, ethyl, propyl, butyl and the isomers thereof; C1-4 alkylene in A¹³ means methylene, ethylene, trimethylene, tetramethylene and the isomers thereof; C1-6 alkylene represented by E¹³ and L¹³ means methylene, ethylene, trimethylene, tetramethylene, pentamethylene, hexamethylene and the isomers thereof; C1-4 alkoxy in M¹³ means methoxy, ethoxy, propoxy, butoxy and the isomers thereof; halogen in M¹³ means chlorine, bromine, fluorine or iodine; the binding position of side chain represented by -O-A¹³ may be any one of 1 to 4 position and more preferred is the 1 position; and the binding position of side chain represented by -E¹³-G¹³-L¹³-M¹³ may be any one of 5 to 8 position and more preferred is the 5 or 6 position.

Moreover, a substance having an EP3 agonist activity includes a compound described in WO99/25358. Preferred as the compound is compounds represented by formula (2-14) 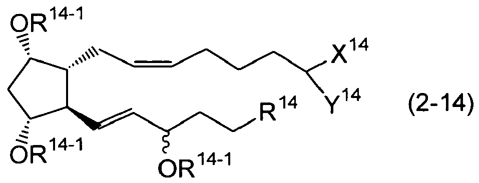 wherein R¹⁴ is substituted heteroaryl having at least two pendant substituents, wherein the pendant substituents are selected from the group consisting of C1-6 alkyl, halogen, trifluoromethyl, COR¹⁴⁻¹, COCF₃, SO₂NR¹⁴⁻¹, NO₂ and CN, or R¹⁴ is substituted heteroaryl having at least one cyano;
R¹⁴⁻¹ is a hydrogen atom or lower alkyl having up to 6 carbon atoms;
X¹⁴ is selected from the group consisting of -OR¹⁴⁻¹ and -N(R¹⁴⁻¹)₂; and
Y¹⁴ is =O or two hydrogen, or salts thereof.

Moreover, a substance having an EP3 agonist activity includes a compound described in JP-A-11-012249. Preferred as the compound is compounds represented by formula (2-15) 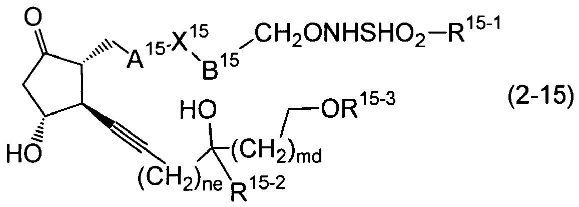 wherein A¹⁵ is a group represented by formula 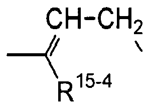 wherein R¹⁵⁻⁴ is a hydrogen atom, C1-4 alkyl or a halogen atom, or a group represented by formula 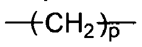 wherein p is 0 or an integer of 1 to 3;
B¹⁵ is a group represented by formula 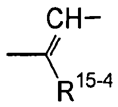 wherein R¹⁵⁻⁴ has the same meaning as described above, or a group represented by formula 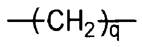 wherein q is an integer of 1 to 4;
X¹⁵ is methylene, oxygen or sulfur;
R¹⁵⁻¹ is C1-4 alkyl, phenyl or phenyl substituted by C1-4 alkyl, C1-4 alkoxy, a halogen atom or C2-5 alkanoyl;
R¹⁵⁻² is a hydrogen atom or C1-4 alkyl;
R¹⁵⁻³ is C1-4 alkyl, phenyl or benzyl, and
ne and md are each independently 0 or 1, or salts thereof.

Moreover, a substance having an EP3 agonist activity includes a compound described in JP-A-10-168056. Preferred as the compound is compounds represented by formula (2-16) 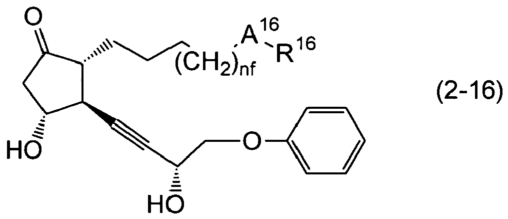 wherein A¹⁶ is ethylene, vinylene or ethynylene;
R¹⁶ is a group represented by formula 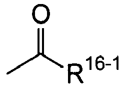 wherein R¹⁶⁻¹ is C1-4 alkyl or C3-8 cycloalkyl, or a group represented by formula 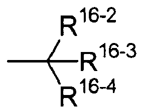 wherein R¹⁶⁻² and R¹⁶⁻³, which are same or different, are a hydrogen atom or C1-4 alkyl, R¹⁶⁻⁴ is C1-4 alkyl, C3-8 cycloalkyl, C1-4 alkoxy, C3-8 cycloalkoxy, hydroxy, C1-4 hydroxyalkyl, C2-8 acyloxy, C1-4 alkylthio, C1-4 alkylsulfinyl, nitro or acetylamino; and
nf is 0 or 1, or salts thereof.

Moreover, a substance having an EP3 agonist activity includes a compound described in JP-A-7-233145. Preferred as the compound is compounds represented by formula (2-17) 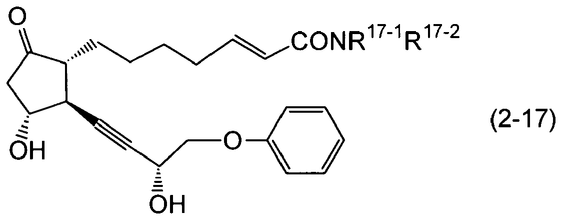 wherein R¹⁷⁻¹ and R¹⁷⁻², which are same or different, are a hydrogen atom, C1-6 alkyl, C3-8 cycloalkyl, methyl substituted by C3-8 cycloalkyl, the monovalent group of C7-12 bridge cyclic hydrocarbon, C1-6 alkylsulfonyl or methoxycarbonylmethyl, or R¹⁷⁻¹ and R¹⁷⁻² are taken together with the nitrogen atom to which they are attached to form the monovalent group of heterocyclic compound, or salts thereof.

Moreover, a substance having an EP3 agonist activity includes a compound described in US4,692,464. Preferred as the compound is compounds represented by formula (2-18) 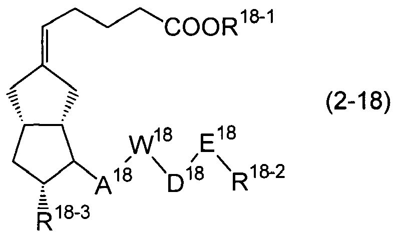 wherein R¹⁸⁻¹ is a hydrogen atom or C1-4 alkyl;
A¹⁸ is trans-CH=CH-;
W¹⁸ is hydroxymethyl optionally protected by tetrahydropyranyl;
D¹⁸ is straight or branched chain having 1 to 5 carbon atoms;
E¹⁸ is -C≡C-;
R¹⁸⁻² is C1-2 alkyl; and
R¹⁸⁻³ is hydroxy optionally protected by tetrahydropyranyl, or salts thereof.

In compounds represented by formula (2-18), more preferred is, for example, (1 S,5S,6R,7R)-5-[7-hydroxy-6-[3(S)-hydroxy-3-methyl-1 (E)-octenyl]bicyclo[3.3.0]oct-2-en-3-yl]pentanoic acid (the compound is called TEI-3356 too.) or an salt thereof.

Moreover, a substance having an EP3 agonist activity includes a compound described in JP-A-51-125255. Preferred as the compound is compounds represented by formula (2-19) 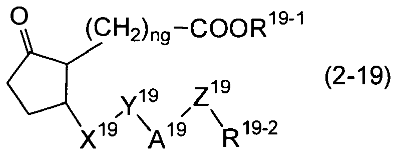 wherein R¹⁹⁻¹ is a hydrogen atom or C1-12 straight or branched alkyl;
R¹⁹⁻² is aryl or heterocycle, which are substituted by one or more substituents selected from a halogen atom, C1-4 straight or branched alkyl, trihalomethyl, C2-4 alkenyl, phenyl, C1-4 alkoxy, hydroxy, nitro, cyano, carboxy, alkocarbonyl having C1-4 alkoxy moiety, hydroxymethylene, alkoxymethylene having C1-4 alkoxy moiety, sulfino, alkylsulfonyl having C1-4 alkyl moiety and sulfamoyl, carbamoyl, N-aminocarbamoyl, amidino, amino and hydroxyimino wherein each said group including nitrogen is optionally substituted by one or more C1-4 alkyl;
ng is an integer of 5 to 8; and
(1) A¹⁹ is C1-12 straight or branched alkylene, X¹⁹ is ethylene or trans-vinylene, Y¹⁹ is carbonyl or -CH(OR¹⁹⁻³)- wherein R¹⁹⁻³ is hydrogen or carboxylic acyl, Z¹⁹ is a bond, an oxygen atom or a sulfur atom, or
(2) A¹⁹ and Z¹⁹ are a bond, X¹⁹ and Y¹⁹ are simultaneously ethylene and carbonyl, trans-vinylene and carbonyl or ethylene and -CH(OR¹⁹⁻³)- wherein R¹⁹⁻³ has the same meaning as described above, or salts thereof.

In compounds represented by formula (2-19), more preferred is (+/-)-15α-hydroxy-9-oxo-16-phenoxy-17,18,19,20-tetranorprosta-13-trans-enoic acid (the compound is called M&B28767 too.) or a salt thereof.

Moreover, a substance having an EP3 agonist activity includes a compound described in JP61-249951. Preferred as the compound is compounds represented by formula (2-20) 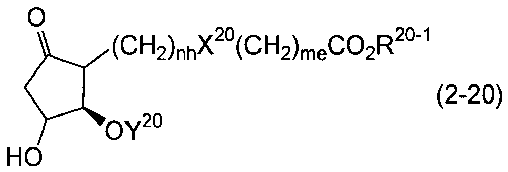 wherein nh is 1 or 2;
me is an integer of 2 to 5 and X²⁰ is cis or trans -CH=CH- or -CH₂-CH₂-, or
me is an integer of 1 to 4 and X²⁰ is -CH=C=CH-;
R²⁰⁻¹ is (1) phenyl which is optionally substituted with C1-4 alkyl, C1-4 alkoxy, C1-4 alkanoyl, methylthio, methylsulfinyl, methylsulfonyl, a halogen atom, -CO₂R²⁰⁻² wherein R²⁰⁻² is a hydrogen atom, C1-4 alkyl or phenyl, - NHCOR²⁰⁻² wherein R²⁰⁻² has the same meaning as described above or is optionally substituted with hydroxy, CH₃CONH- or benzoylamino, -CONR²⁰⁻³R²⁰⁻⁴ wherein R²⁰⁻³ or R²⁰⁻⁴ which are same or different, are each independently a hydrogen atom or C1-4 alkyl, -NHCONH₂, -CH₂CH(CONH₂)NHCOCH₃ or 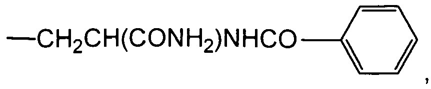 or (2) 2-naphthyl; and
Y²⁰ is 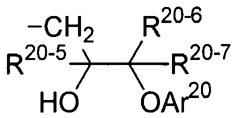 wherein R²⁰⁻⁵, R²⁰⁻⁶ and R²⁰⁻⁷ are each independently a hydrogen atom or methyl, and at least one of the above is a hydrogen atom, and Ar²⁰ is phenyl optionally substituted by one or two substituents selected from C1-4 alkyl, C1-4 alkoxy, C1-4 alkylthio, C1-4 alkylsulfinyl, C1-4 alkylsulfonyl, a halogen atom or trifluoromethyl, or salts thereof.

In compound represented by formula (2-20), more preferred is, for example, (-)-[1(R)-[1α (Z),2β(R*),3α]]-7-[3-hydroxy-2-(2-hydroxy-3-phenoxypropoxy)-5-oxocyclopentyl]-4-heptenoic acid 4-(benzoylamino)phenyl ester (the compound is called GR63799X.) or a salt thereof.

Moreover, a substance having an EP3 agonist activity includes a compound described in US4,863,961. Preferred as the compound is compounds represented by formula (2-22) 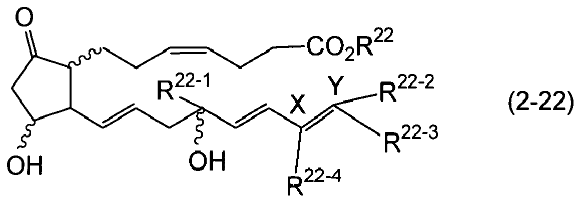 wherein R²² is a hydrogen atom or C1-4 alkyl;
R²²⁻¹ is a hydrogen atom, vinyl or C1-4 alkyl;
wavy line is R or S stereochemistry; and
R²²⁻², R²²⁻³ and R²²⁻⁴ are a hydrogen atom or C1-4 alkyl, or
R²²⁻² and R²²⁻³ form a cycloalkenyl having 4 to 6 carbon atoms together with carbon Y, or
R²²⁻² and R²²⁻⁴ form a cycloalkenyl having 4 to 6 carbons together with carbons X and Y, or salts thereof.
In compounds represented by formula (2-22), more preferred is, for example, methyl 7-(2β-(6-(1-cyclopentyl)-4R-hydroxy-4-methyl-1E,5E-hexadienyl)-3α-hydroxy-5-oxo-1R,1α-cyclopentyl)-4Z-heptenoate (the compound is called SC-46275.) or a salt thereof.

Moreover, a substance having an EP3 agonist activity includes a compound described in US3,985,791. Preferred as the compound is compounds represented by formula (2-24) 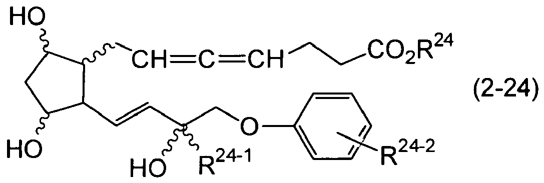 wherein R²⁴ is a hydrogen atom or C1-4 alkyl;
R²⁴⁻¹ is a hydrogen atom, methyl or ethyl; and
R²⁴⁻² is a hydrogen atom, o-, m-, or p-halo (fluoro, chloro or bromo), o-, m- or p-trifluoromethyl, o-, m- or p-lower alkyl or o-, m- or p-lower alkoxy; and
wherein, when R²⁴⁻¹ is α-configuration, the hydroxyl group, attached to the same carbon atom as R²⁴⁻¹ is β-configuration; and when R²⁴⁻¹ is β-configuration, the hydroxyl group, attached to the same carbon atom as R²⁴⁻¹ is α-configuration, or salts thereof.

In compounds represented by formula (2-24), more preferred is, for example, 9-oxo-11 α, 15α-dihydroxy-16-phenoxy-17,18,19,20-tetranorprosta-4,5,13-trans-trienoic acid methyl ester (the compound is called Enprostil too.) or a salt thereof.

Moreover, as a substance having an EP3 agonist activity, more preferred is 16-phenoxy-ω-17,18,19,20-tetranor-PGE2 methylsulfonamide (the compound is called Sulprostone too.) or a salt thereof.

### Processes for the preparation of the compound used in the present invention

The compounds of the presented invention represented by formula (1-1) and salts thereof can be prepared by methods described in the specification of EP860430. Moreover, (5Z,9β,11α,13E)-17,17-propano-17,16-dihydroxy-9-chloro-20-norprosta-5,13-dienoic acid and the pharmaceutically acceptable salt thereof which are more preferable compounds can be prepared by methods described in the specification of JP-A-11-193268 and (5Z,9β,11α,13E)-17,17-propano-11,16-dihydroxy-9-chloroprosta-5,13,19-trienoic acid and the salt thereof can be prepared by methods described in the specification of JP-A-2000-128858.

The compounds of the present invention represented by formula (1-2) and the salt thereof can be prepared by methods described in the specification of WO99/33794.

The compounds of the present invention represented by formula (1-3) and the salts thereof can be prepared by methods described in the specification of EP974580.

The compounds of the present invention represented by formula (1-4) and the salts thereof can be prepared by methods described in the specification of W02003/74483.

The compounds of the present invention represented by formulae (1-5-1) and (1-5-2), trans-2-(4-(1-hydroxyhexyl)phenyl)-5-oxocyclopentaneheptanoic acid which is more preferable compound and, and the salts thereof can be prepared by methods described in the specification of WO95/19964.

The compounds of the present invention represented by formula (1-6), 2-[3-(4-tert-butylbenzyl)-N-(pyridin-3-ylsulfonyl)amino-methyl]phenoxy]acetic acid which is more preferable compound and, and the salts thereof can be prepared by methods described in the specifications of WO98/28264, WO99/19300 and EP0911321.

The compounds of the present invention represented by formula (1-7) and the salts thereof can be prepared by methods described in the specification of WO98/58911.

The compounds of the present invention represented by formulae (1-8-1), (1-8-2) and (1-8-3) and the salts thereof can be prepared by methods described in the specification of US5,698,598.

The compounds of the present invention represented by formula (1-9) and the salts thereof can be prepared by methods described in the specification of US6,376,533.

The compounds of the present invention represented by formula (1-21), [1R[1α,2β(1E,4R*),3α]]-3-hydroxy-2-[4-hydroxy-4-(1-propylcyclobutyl)-1-butenyl]-5-oxocyclopentane-heptanoic acid methyl ester, (2R,3R,4R)-4-hyd roxy-2-(7-hyd roxyheptyl)-3-[(E)-(4RS)-(4-hyd roxy-4-methyl-1-octenyl)]cyclopentanone, which are more preferable compound and, and the salts thereof can be prepared by methods described in the specification of US4,132,738.

The compounds of the present invention represented by formula (1-23), (+/-)-15-deoxy-16-α,β-hydroxy-16-methyl PGE1 methyl ester which is more preferable compound and, and the salts thereof can be prepared by methods described in the specification of US3,965,143.

The compounds of the present invention represented by formula (2-10), 11α,15α-dimethoxy-9-oxoprosta-5Z,13E-dienoic acid which is more preferable compound and, and the salts thereof can be prepared by methods described in the specification of WO98/34916.

The compounds of the present invention represented by formula (2-11) and the salts thereof can be prepared by methods described in the specification of JP-A-7-215929.

The compounds of the present invention represented by formula (2-12), 2-[5-[2-[N-(diphenylmethyl)carbamoyl]ethyl]naphthalen-1-yloxy]acetic acid which is more preferable compound and, and the salts thereof can be prepared by methods described in the specification of JP-A-8-239356.

The compounds of the present invention represented by formula (2-13) and the salts thereof can be prepared by methods described in the specification of WO97/05091.

The compounds of the present invention represented by formula (2-14) and the salts thereof can be prepared by methods described in the specification of WO99/25358.

The compounds of the present invention represented by formula (2-15) and the salts thereof can be prepared by methods described in the specification of JP-A-11-012249.

The compounds of the present invention represented by formula (2-16) and the salts thereof can be prepared by methods described in the specification of JP-A-10-168056.

The compounds of the present invention represented by formula (2-17) and the salts thereof can be prepared by methods described in the specification of JP-A-7-233145.

The compounds of the present invention represented by formula (2-18), 5-[(1S,5S,6R,7R)-7-hydroxy-6-[(E)-(S)-4-hydroxy-4-methyl-1-octenyl]bicyclo[3.3.0]oct-2-en-3-yl]pentanoic acid which is more preferable compound and, and the salts thereof can be prepared by methods described in the specification of US4,692,464.

The compounds of the present invention represented by formula (2-19), (+/-)-15α-hydroxy-9-oxo-16-phenoxy-17,18,19,20-tetranorprosta-13-trans-enoic acid which is more preferable compound and, and the salts thereof can be prepared by methods described in the specification of JP-B-51-125255.

The compounds of the present invention represented by formula (2-20), [1R-[1α(Z),2β(R*),3α]]-4-(benzoylamino)phenyl-7-[3-hydroxy-2-(2-hydroxy-3-phenoxypropoxy)-5-oxocyclopentyl]-4-heptenoic acid which is more preferable compound and, and the salts thereof can be prepared by methods described in the specification of JP-A-61-249951.

The compounds of the present invention represented by formula (2-22), methyl 7-(2β-(6-(1-cyclopentyl)-4R-hydroxy-4-methyl-1E,5E-hexadienyl)-3α-hydroxy-5-oxo-1R,1α-cyclopentyl)-4Z-heptenoate which is more preferable compound and, and the salts thereof can be prepared by methods described in the specification of US4,863,961.

The compounds of the present invention represented by formula (2-24), 9-oxo-11α,15α-dihydroxy-16-phenoxy-17,18,19,20-tetranorprosta-4,5,13-transtrienoic acid methyl ester which is more preferable compound and, and the salts thereof can be prepared by methods described in the specification of US3,985,791.

### Substance screening method of the invention

The invention provides a method for screening a substance which has EP2 and/or EP3 agonist activity having the effect of stimulating chondrogenesis, stimulating chondrocyte growth, stimulating chondrocyte differentiation, inhibiting cartilage calcification or inhibiting cartilage degradation and also having cartilage disorder treating effect.

A series of actions by the substance having EP2 and/or EP3 agonist activity of the invention are related to a group of genes which are essential for the expression of the actions or controlled together with the expression of actions. Particularly, at least stimulation of fibronectin mRNA expression, expression of fibronectin mRNA, expression of cyclin D1 mRNA, expression of myc-associated zinc finger protein (MAZ) mRNA, expression of AP2α mRNA or expression of 14-4-3γ mRNA, or inhibition of the expression of osteopontin mRNA, is strongly correlated with the aforementioned actions by the substance having EP2 and/or EP3 agonist activity, and these actions are generated via or together with the expression control of these mRNA species. Accordingly, the substance which has EP2 and/or EP3 agonist activity having the effect of stimulating chondrogenesis, stimulating chondrocyte growth, stimulating chondrocyte differentiation, inhibiting cartilage calcification or inhibiting cartilage degradation and also having cartilage disorder treating effect can be screened by measuring induction or inhibition of mRNA expression by substances to be tested in chondrocytes or cell strains. The measurement can be carried out in accordance with a reporter gene assay (e.g., a luciferase assay, a β-galactosidase assay, a DFP assay, or an SEAP assay), a DNA microarray method, an RT-PCR method or a northern blotting method or corresponding methods thereof. The DNA microarray method can be carried out using a commercially available cDNA chip, and preferably, those which are prepared from a cDNA of an optionally selected cartilage tissue-related gene can be used. Alternatively, it can also be carried out by measuring produced amount of an expression induced protein such as an intracellular protein, a cell surface protein or a secretory protein by the conventional method such as an immunological detection method, a method employing a chromatography or the like.

The effect of stimulating chondrocyte growth by the substance of the invention having EP2 and/or EP3 agonist activity can be evaluated by the method which measures reinforcement of the growth activity of a chondrocyte or a cell strain thereof by the substance having EP2 and/or EP3 agonist activity. The measurement can be carried out, for example, by a method employing a hemocytometer, a method employing a cell counter or FACS, a method employing H thymidine or the like radioisotope, a bromouracil (to be referred to as BrU hereinafter) incorporation method, an LDH (lactate dehydrogenase) method, a Neutral Red or Crystal Violet staining method, a method employing a tetrazolium salt (e.g., WST-8, MTT or XTT). Respective methods can be carried out by conventional methods or in accordance with the instructions attached to commercially available assay kits.

The effect of stimulating chondrogenesis by the substance of the invention having EP2 and/or EP3 agonist activity can be evaluated by the histological analysis of articular cartilage tissue of epiphysis region. Illustratively, actions of the substances can be evaluated using a mammal model in which a part of an epiphysial articular cartilage is broken or damaged artificially or by a cartilage disorder, by topically administering the substance having EP2 and/or EP3 agonist activity to the broken or damaged region. The evaluation object of this case is histological findings of regenerated cartilage tissue or the area ratio thereof. In this connection, a device capable of partially breaking the cartilage layer alone without giving damage to the cartilage lower bone can be used in the artificial chondral defect.

The effect of inhibiting cartilage calcification by the substance of the invention having EP2 and/or EP3 agonist activity can be evaluated by measuring calcification rate of cartilage tissue. Illustratively, bone labeling is carried out by administering calcein chelating calcium which is the main component of deposition minerals, and calcification rate during administration intervals of calcein is calculated.

### Cartilage grafts of the invention

Cartilage grafts as used herein means primary-cultured chondrocytes, chondrocyte strains or cartilages regenerated in vitro. These can be used as safe cartilage grafts for, for example, rheumatoid arthritis, osteoporosis, osteoarthritis, osteochondral defect, cartilage damage, articular disk damage, meniscus injury, chondrodysplasia, incomplete repair and healing of bone fracture, refracture, achondroplasia, achondrogenesis, bone deformation or spondylosis deformans, dyschondrogenesis, chondrodystrophia, articular chondrocalcinosis, acute purulent arthritis, tuberculous arthritis, syphilitic arthritis, systemic lupus erythematosus, spondylosis deformans, disk herniation, injury by sports, keypuncher's disease, osteosarcoma, myeloma, osteomalacia, rickets, osteitis fibrosa, renal ostaodystrophy and bone Behcet disease, which are known as various diseases caused by cartilage disorders. In addition, the cartilage-related disease treating agent which comprises a substance having EP2 and/or EP3 agonist activity as the active ingredient can also be used as a chondrocyte culture agent for the production of cartilage grafts. This is the use of the effect of stimulating chondrogenesis, stimulating chondrocyte growth, stimulating chondrocyte differentiation, inhibiting cartilage calcification and inhibiting cartilage degradation of the substance having EP2 and/or EP3 agonist activity for the in vitro production of cartilage grafts.

It is also possible to use the primary-cultured chondrocyte or chondrocyte strain of the invention only for the cartilage transplantation.

Illustratively, cartilage regeneration can be quickened by culturing a cartilage tissue collected from a patient or a mesenchymal stem cell collected from the bone marrow of the patient or the like, and transplanting the same into affected part tissues of the aforementioned diseases. In this case, since the number of collectable cartilage tissues or of mesenchymal stem cells contained in the bone marrow is limited, their efficient differentiation or proliferation by in vitro culturing becomes the problem. In order to prepare a transplantable cartilage tissue or chondrocyte, the substance of the invention can be used for stimulating regeneration of the same tissue or differentiation and proliferation of the same cell.

The primary-cultured chondrocyte or chondrocyte strain to be used in the invention may be either a clonal or a polyclonal cell with the proviso that it is a cell which can be differentiated into a chondrocyte. For example, a bone marrow derived cell, an articular cartilage derived cell, a skin derived cell, a reproductive cell, fetus derived cell and the like can be used, and they are illustratively a h mesenchymal stem cell and a dedifferentiated human chondrocyte, more illustratively the cell strain of the invention recognized by the international deposition number FERM BP-10029. This cell strain has been deposited on June 12, 2003, in International Patent Organism Depositary, National Institute of Advanced Industrial Science and Technology, Central 6, 1-1-1 Higashi, Tsukuba-shi, Ibaraki, Japan (postal code 305-8566), (deposition number FERM P-19393), and transferred to the international deposition on May 27, 2004 (international deposition number FERM BP-10029).

The primary-cultured chondrocyte or chondrocyte strain of the invention can be used for the screening or the like of a new gene concerned in the stimulating chondrogenesis, stimulating chondrocyte growth, stimulating chondrocyte differentiation or chondrocyte differentiation acceleration or various diseases caused by cartilage disorders. The primary-cultured chondrocyte or chondrocyte strain which can be used for the above purpose can be isolated or prepared from the tissues of the primates including guinea pig, rat, mouse, domestic fowl, rabbit, pig, sheep, cattle, horse, monkey and human.

### Application to pharmaceuticals

The compounds of the present invention include salts prepared at the known methods. Pharmacologically acceptable salts are preferred. It has been confirmed that the compounds of the present invention have low toxicity so that it is possible to allow it by pharmacology and are sufficiently safe for use as pharmaceutical preparations.

Said pharmacologically acceptable salt is salt of alkali metal, salt of alkaline earth metal, ammonium salt or amine salt etc., when the parent compound is the acidic compound. On the other hand, when the parent compound is the basic compound, the salt is organic or inorganic acid addition salt etc.

The pharmacologically acceptable salt is preferably water-soluble. The suitable salt means, for example, salt of alkali metal (potassium, sodium etc.), salt of alkaline earth metal (calcium, magnesium, etc.), ammonium salt, pharmaceutically acceptable salt of organic amine and amino acid (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, etc.).

The acid addition salt is preferably water-soluble. The suitable acid addition salt means, for example, inorganic acid salt (hydrochloride, hydrobromate, hydroiodate, sulfate, phosphate, nitrate, etc.), or organic acid salt (acetate, lactate, tartrate, benzoate, citrate, methane sulfonate, ethane sulfonate, benzene sulfonate, toluene sulfonate, isethionate, glucuronate, gluconate, etc.), etc.

In addition, the compound of the present invention and the salt thereof may be converted solvate.

The solvate is preferably non toxic and water-soluble. The suitable solvate is, for example, solvate of water or alcohol (e.g. ethanol).

Moreover, the compounds used in the present invention may be prodrugs thereof prepared by known methods.

A prodrug of the compounds used in the present invention mean a compound which is converted to the compound used in the present invention by reaction with an enzyme, gastric acid or the like in the living body. For example, with regard to a prodrug of the compound used in the present invention, when the compound used in the present invention has a hydroxyl group, compounds where the hydroxyl group is, for example, acylated, alkylated, phosphorylated or borated (e.g., compounds in which the hydroxyl group of the compound used in the present invention is acetylated, palmitoylated, pivaloylated, succinylated, fumarylated, alanylated or dimethylaminomethylcarbonylated); and that the carboxyl group of the compound used in the present invention is, for example, esterified or amidated (e.g., compounds in which the carboxyl group of the compound used in the present invention is made into ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methylamide). Those compounds may be produced by a known method per se. The prodrug of the compound used in the present invention may be either a hydrate or a non-hydrate.

The compounds used in the present invention or the esters thereof may be converted into the corresponding cyclodextrin clathrates by the method described in the specification of GB1,351,238 or GB1,419,221 using α-, β- or γ-cyclodextrin or a mixture thereof. Converting into the corresponding cyclodextrin clathrates serves to increase the stability and solubility in water of the compounds, and therefore it is useful in the use for pharmaceuticals.

The remedies of the present invention are normally administered to the entire or local part of human body orally or parenterally.

The doses to be administered are determined depending upon, for example, age, body weight, symptom, the desired therapeutic effect, the route of administration, and the duration of the treatment as well as the medicament used in the invention. In the human adult, the doses per person are generally from 1 µg to 100 mg, by oral administration, up to several times per day, and from 0.1 ng to 10 mg, by parenteral administration, up to several times per day. Among the parenteral administration, preferred is continuous administration from 1 to 24 hours per day from vein.

As mentioned above, the doses depend upon various conditions. Therefore, there are cases in which doses lower than or greater than the ranges specified above may be used.

The remedies of the present invention may be administered in the composition of, for example, solid compositions or liquid compositions, each for oral administration, or injections, external use, suppositories, inhalant or nasal spray each for parenteral administration.

Examples of the solid preparations for internal use for oral administration include tablets, pills, capsules, powders, granules and the like. The capsules include hard capsules and soft capsules. The tablets include sublingual tablets, intraoral patches, orally fast disintegrating tablets and the like.

Such a solid preparation for internal use is prepared by a formulation method commonly employed by using one or two or more active substances either as it is or as a mixture with an excipient (lactose, mannitol, glucose, microcrystalline cellulose, starch, etc.), a binder (hydroxypropylcellulose, polyvinylpyrrolidone, magnesium metasilicate aluminate, etc.), a disintegrating agent (calcium cellulose glycolate, etc.), a lubricant (magnesium stearate, etc.), a stabilizer and a solubilization agent (glutamic acid, aspartic acid, etc.). If necessary, it may be coated with a coating agent (sucrose, gelatin, hydroxypropylcellulose, hydroxypropylmethylcellulose phthalate, etc.). It may be coated with two or more layers. Moreover, capsules made of an absorbable material such as gelatin are involved in the scope thereof.

The sublingual tablets may be prepared in accordance with a well known method. For example, a sublingual tablet is prepared by a formulation method commonly employed by using one or more active substances are used mixed with an excipient (lactose, mannitol, glucose, microcrystalline cellulose, starch, etc.), a binder (hydroxypropylcellulose, polyvinylpyrrolidone, magnesium metasilicate aluminate, etc.), a disintegrator (starch, L-hydroxypropyl cellulose, carboxymethyl cellulose, croscarmellose sodium, calcium cellulose glycolate, etc.), a lubricant (magnesium stearate, etc.), a swelling agent (hydroxypropyl cellulose, hydroxylpropylmethy cellulose, carbopol, carboxymethyl cellulose, polyvinyl alcohol, xanthan gum, guar gum, etc.), a swelling aid agent (glucose, fructose, mannitol, xylitol, erythritol, maltose, trehalose, phosphate, citrate, silicate, glycine, glutamic acid, arginine, etc.), a stabilizer and a dissolution aid (polyethylene glycol, propylene glycol, glutamic acid, aspartic acid, etc.), a flavoring agent (orange, strawberry, mint, lemon, vanilla, etc.). If necessary, it may be coated with a coating agent (sucrose, gelatin, hydroxypropylcellulose, hydroxypropylmethylcellulose phthalate, etc.). If necessary, it may be coated with two or more layers. Moreover, it may also further comprise some additives such as sweetening agents, antioxidants, coloring agents, preservatives and the like.

The intraoral patch may be prepared in accordance with a well known method. For example, a intraoral patch is prepared by a formulation method commonly employed by using one or more active substances are used mixed with an excipient (lactose, mannitol, glucose, microcrystalline cellulose, starch, etc.), a binder (hydroxypropylcellulose, polyvinylpyrrolidone, magnesium metasilicate aluminate, etc.), a disintegrator (starch, L-hydroxypropyl cellulose, carboxymethyl cellulose, croscarmellose sodium, calcium cellulose glycolate, etc.), a lubricant (magnesium stearate, etc.), a attach agent (hydroxypropyl cellulose, hydroxylpropylmethy cellulose, carbopol, carboxymethyl cellulose, polyvinyl alcohol, xanthan gum, guar gum, etc.), a attach aid agent (glucose, fructose, mannitol, xylitol, erythritol, maltose, trehalose, phosphate, citrate, silicate, glycine, glutamic acid, arginine, etc.), a stabilizer and a dissolution aid (polyethylene glycol, propylene glycol, glutamic acid, aspartic acid, etc.), a flavoring agent (orange, strawberry, mint, lemon, vanilla, etc.) and the like. If necessary, it may be coated with a coating agent (sucrose, gelatin, hydroxypropylcellulose, hydroxypropylmethylcellulose phthalate, etc.) and the like. If necessary, it may be coated with two or more layers. Moreover, it may also further comprise some additives such as sweetening agents, antioxidants, coloring agents, preservatives and the like.

Liquid forms for oral administration include pharmaceutically acceptable solutions, suspensions and emulsions, syrups and elixirs. In such forms, one or more of the active compound(s) may be dissolved, suspended or emulized into diluent(s) commonly used in the art (such as purified water, ethanol or a mixture thereof). Besides such liquid forms may also comprise some additives, such as wetting agents, suspending agents, emulsifying agents, sweetening agents, flavoring agents, aroma, preservative or buffering agent.

In the parenteral administration, formulation of external use include, for example, ointment, ger, cream, poultice, patch, liniment, atomized agent, inhalation, spray, aerosol, eye drops and nasal spray, etc. They includes one or more of the active compound(s) and be prepared by known method or usual method.

Ointment is prepared by known method or usual method. For example, it is prepared by levigation or fusion of one or more of the active compound(s) and substrate. The substrate of ointment is selected from known or usual one. For example, higher fatty acid or higher fatty acid ester (adipic acid, myristic acid, palmitic acid, stearic acid, oleic acid, adipic acid ester, myristic acid ester, palmitic acid ester, stearic acid ester, oleic acid ester, etc.), wax (yellow beeswax, Spermaceti, ceresin, etc.), surfactant (polyoxyethylene alkyl ether phosphoric acid ester, etc.), higher alcohol (cetanol, stearil alcohol, cetostearyl alcohol, etc.), silicon oil (dimethyl polysiloxane, etc.), hydrocarbon (hydrophilic petrolatum, white petrolatum, purified lanolin, light liquid paraffin, etc.), glycol (ethylene glycol, diethylene glycol, propylene glycol, polyethylene glycol, macrogol, etc.), vegetable oil (castor oil, olive oil, sesame oil, turpentine oil, etc.), animal oil (mink oil, egg yolk oil, squalane, squalene, etc.), water, absorption accelerator, skin fit inhibitor, etc. are used as single substance selected from them or mixture which consists of two or more kinds that is selected from them. Moreover, humectant, preservative agent, stabilizer, antioxidative agent, fragrant materials, etc. may be contained.

Ger is prepared by known method or usual method. For example, it is prepared by fusion of one or more of the active compound(s) and substrate. The substrate of gel is selected from known or usual one. For example, lower alcohol (ethanol, isopropylalcohol, etc.), gelling agent (carboxy methyl cellulose, hydroxy ethyl cellulose, hydroxy propyl cellulose, ethyl cellulose, etc.), neutralizing agent, (triethanolamine, diisopropanolamine, etc.), surfactant, (polyethylene glycol monostearate, etc.), gum, water, absorption accelerator, skin fit inhibitor, etc. are used as single substance selected from them or mixture which consists of two or more kinds that is selected from them. Moreover, preservative agent, antioxidative agent, fragrant materials, etc. may be contained.

Cream is prepared by known method or usual method. For example, it is prepared by fusion or emulsification of one or more of the active compound(s) and substrate. The substrate of cream is selected from known or usual one. For example, higher fatty acid ester, lower alcohol, hydrocarbon, polyalcohol (propylene glycol, 1,3-butylene glycol, etc.), higher alcohol (2-hexyldecanol, cetanol, etc.), emulsifying agent (polyoxyethylene alkyl ether, fatty acid ester, etc.), water, absorption accelerator, skin fit inhibitor, etc. are used as single substance selected from them or mixture which consists of two or more kinds that is selected from them. Moreover, preservative agent, antioxidative agent, fragrant materials, etc. may be contained.

Poultice is prepared by known method or usual method. For example, it is prepared by fusion of one or more of the active compound(s) and substrate, and then the kneaded one is laid over support medium. The substrate for poultice is selected from known or usual one. For example, thickening agent (polyacrylic acid, polyvinylpyrolidone, gum acacia, starch, gelatin, methyl cellulose, etc.), bulking agent (kaolin, zinc oxide, talc, calcium, magnesium, etc.), water, solubilizing agent, thickener, skin fit inhibitor, etc. are used as single substance selected from them or mixture which consists of two or more kinds that is selected from them. Moreover, preservative agent, antioxidative agent, fragrant materials, etc. may be contained.

Patch is prepared by known method or usual method. For example, it is prepared by fusion of one or more of the active compound(s) and substrate, and then laid over support medium. The substrate for patch is selected from known or usual one. For example, polymer substrate, fat, higher fatty acid, thickener, skin fit inhibitor, etc. are used as single substance selected from them or mixture which consists of two or more kinds that is selected from them. Moreover, preservative agent, antioxidative agent, fragrant materials, etc. may be contained.

Liniment is prepared by known method or usual method. For example, one or more of the active compound(s) may be dissolved, suspended or emulsified in water, alcohol (ethanol, polyethylene glycol, etc.), higher fatty acid, glycerin, soap, emulsifying agent, suspending agent, etc. as single substance selected from them or mixture which consists of two or more kinds that is selected from them. Moreover, preservative agent, antioxidative agent, fragrant materials, etc. may be contained.

Atomized agent, inhalation and spray may comprise in addition to a diluent, a stabilizer such as sodium bisulfite and an isotonization buffer such as sodium chloride, sodium citrate or citric acid. The preparation process of sprays is described in detail in, for example, U.S. Patent Nos. 2,868,691 and 3,095,355.

Injections for parenteral administration include sterile aqueous, suspensions, emulsions and solid forms which are dissolved or suspended into solvent(s) for injection immediately before use. In injections, one or more of the active compound(s) may be dissolved, suspended or emulized into solvent(s). The solvents may include distilled water for injection, physiological salt solution, vegetable oil, propylene glycol, polyethylene glycol, alcohol, e.g. ethanol, or a mixture thereof. Injections may comprise some additives, such as stabilizing agents, solution adjuvants (such as glutamic acid, aspartic acid or POLYSORBATE80 (registered trade mark)), suspending agents, emulsifying agents, soothing agent, buffering agents, preservative.
They may be sterilized at a final step, or may be prepared by an aseptic manipulation . They may also be manufactured in the form of sterile solid forms, for example, freeze-dried products, which may be dissolved in sterile water or some other sterile diluent(s) for injection immediately before use.

The dosage of inhalations for parenreral administration include aerosol, powders for inhalation or liquids for inhalation. The liquids for inhalation may be dissolved or suspended in water or the other appropriate solvent as needed.

Such inhalations are prepared in a known method.

For example, a liquid for inhalation is prepared by selecting proper additives from an antiseptic (such as benzalkonium chloride or p-aminobenzonic acid), a coloring agent, a buffering agent (such as sodium phosphate or sodium acetate), an isotonizing agent (such as sodium chloride or concentrated glycerin), thickening agent (such as carboxyvinylpolymer), or an accelerator of absorption, etc., if necessary.

A powder for inhalation is prepared by selecting proper additives from a lubricant agent (such as stearin acid and the salt thereof), a binding agent, (such as starch, dextrin), a diluting agent (such as lactose, cellulose), a coloring agent, an antiseptic (such as benzalkonium chloride or p-aminobenzonic acid), an accelerator of absorption, etc., if necessary.

In case of administration of liquid for inhalation, spray (atomizer, nebulizer) is usually used and in case of administration of powder for inhalation, inhalation administration apparatus for powder agents is usually used.

The other compositions for parenteral administration include suppositories for intrarectal administration and pessaries for vaginal administration which comprise one or more of the active substance(s) and may be prepared by methods known per se.

The depot preparation is not limited to its form so far as the compound described in the present invention can be continuously administered to site of disease. The extended-release preparation may be in the form of, e.g., embedding preparation.

Examples of a bioabsorbable polymer enployed in the film of the depot film preparation of the remedy of the present invention include aliphatic acid ester polymers and copolymers thereof, polyacrylic acid esters, polyhydroxybutyric acids, polyalkylene oxalates, polyorthoesters, polycarbonates, and polyaminoacids. These compounds may be used singly or in admixture of two or more thereof. Examples of the aliphatic acid ester polymers and copolymers thereof include polylactic acid, polyglycolic acid, polycitric acid, polymalic acid, and lactic acid-glycolic acid copolymer. These compounds may be used singly or in admixture of two or more thereof. Besides these compounds, poly-α-cyanoacrylic acid esters, poly-β-hydroxybutyric acids, polytrimethyleneoxates, polyorthoesters, polyorthocarbonates, polyethylene carbonates, poly-γ-benzyl-L-glutamic acids and poly-L-alanines may be used singly or in admixture of two or more thereof. Preferred among these compounds are polylactic acids, polyglycolic acids or lactic acid-glycolic acid copolymers.

Lactic acid used in polylactic acids or lactic acid-glycolic acid copolymers includes L-lactic acid or DL-lactic acid

The average molecular weight of these bioabsorbable polymers to be used in the present invention is preferably from about 2,000 to 800,000, more preferably from about 5,000 to 200,000. For example, the polylactic acid preferably has a weight-average molecular weight of from about 5,000 to 100,000, more preferably from about 6,000 to 50,000. The polylactic acid can be synthesized according to any known preparation method per se.

In the lactic acid-glycolic cid copolymer, the composition ratio of the lactic acid to the glycolic acid is preferably from about 100/0 to 0/100 (w/w), particularly from about 90/10 to 30/70. The weight-average molecular weight of the lactic acid-glycolic acid copolymer is preferably from about 5,000 to 100,000, more preferably from about 10,000 to 80,000. The lactic acid-glycolic acid copolymer can be synthesized according to any known preparation method per se.

A method of preparation of the film preparation is not limited. The film preparation can be prepared by, for example, a method to prepare film-like material by dissolving the aforementioned bioabsorbable polymer and an active compound of the present invention in an organic solvent, and then subjecting the solution to distillation to dryness, air drying or freeze dry; a method with dissolving bioabsorbable polymer in an organic solvent and dissolving an active compound in water or the organic solvent which cannot be mixed with the aforementioned solvent, and then emulsifying and freeze-drying; or a method with gelling material obtained by dissolving the aforementioned bioabsorbable polymer and a compound used in the present invention in a proper solvent, and then adding a granulating agent (e.g., cellulose, polycarbonate) to the solution.

The remedy of the present invention can be used for the treatment of cartilage-related diseases and the like because the compound described in the present invention can be gradually released normally for 1 week to 3 months, though depending on the kind and added amount of the bioabsorbable polymer. Among these, especially in the case of the patient who has the aforementioned diseases, it is often required that the affected part be fixed and covered with a plaster bandage. Accordingly, continuous acceleration of treatment by once administration rather than frequent administration is required. Thus, the remedy of the present invention is useful particularly in this treatment.

The dose of the remedy of the present invention depends on the duration of release of pharmaceutical preparations, the animal to be administered, etc., but may be the effective amount of the compound used in the present invention. When administered to fracture as a film preparation, for example, one time dose for adult (weight: 50 kg) is from about 0.001 mg to 500 mg, preferably from about 0.01 mg to 50 mg as calculated in terms of effective component. The medicament of the present invention may be administered once 1 week to 3 months in the aforementioned amount.

The remedy of the present invention may be administered as a combined preparation by combining with other medicaments for the purpose of supplementing and/or enhancing of prevention and/or treatment effect of the compound; improvement in pharmacokinetics and absorption and reduction of dose of the compound; and/or reduction of side effect of the compound.

Specially, it may be used with medicaments for treating other bone diseases. The combined medicaments include, for example, antiinflammatory steroids (for example, prednisolone, hydrocortisone, methylprednisolone, dexamethasone, betamethasone etc.) nonsteroidal anti-inflammatory drug (for example, indometacin, diclofenac, loxoprofen, ibuprofen, aspirin, piroxicam, sulindac), hyaluronic acid preparation (for example, sodium hyaluronate), or growth factor of chondrocyte (for example, transforming growth factor-β (TGF-β), insulin like growth factor (IGF-I), basic fibroblast growth factor (bFGF), combination of epidermal growth factor (EGF) and insulin, growth factor, or platelet-derived growth factor (PDGF). Herein, two or more of the aforementioned other medicaments may be administered in combination with each other.

In addition, the remedy of the present invention may be administered in combination with cartilage grafts or chondrocyte for transplant. As the method of administration, it is desirable to administer the depot preparation, for example, the depot film preparation.

The combined preparation of the remedies of the present invention with other medicaments may be administered in a form of a compounded agent in which both components are compounded in a preparation or may be in a form in which they are administered by means of separate preparations. The case of administration by means of separate preparations includes a simultaneous administration and administrations with time difference. In the case of administrations with time difference, the medicament of the present invention may be firstly administered followed by administering the other medicament or the other medicament may be administered firstly followed by administering the medicament of the present invention. Methods for each of the administration are the same or different.

The amount used of the remedy of the present invention and the other medicament is not especially limited. If it is an amount safely used, any amount is acceptable. Moreover, examples of the other medicaments for supplementing and/or enhancing the treatment effect of the medicaments of the present invention include not only known compounds but also new compound.

The other medicament may be any preparation generally used. For example, solid compositions (tablets, pills, capsules, dispersible powders and granules etc.) and liquid compositions (solutions, suspensions, emulsions, syrups and elixirs etc.) etc. are included.

### The effect of the present invention

The present invention provides a remedy for cartilage-related diseases containing as the active ingredient a substance having an EP2 and/or EP3 agonist activity. A substance having an EP2 and/or EP3 agonist activity has one or more effects selected from promoting chondrogenesis, promoting chondrocyte growth, promoting chondrocyte differentiation, inhibiting cartilage calcification and inhibiting cartilage degradation, and, therefore, is useful as a remedy for cartilage-related diseases.

### BRIEF DESCRIPTION OF THE DRAWINGS

Fig. 1 shows each EP expression in cartilage tissue or cell. Fig. 1 (a) is an in situ hybridization image of each EP expression in a newborn mouse tibial epiphysial cartilage. Fig. 1 (b) shows results of RT-PCR, and 1 is the result on human articular cartilage tissue, 2 is that on human articular cartilage primary-cultured cell, and 3 is that on each EP expression-positive tissue.
Fig. 2 shows an expression of cartilage-related genes in a p53 defective mouse articular cartilage derived cell. Fig. 2 (a) and (b) show a result of RT-PCR of cartilage-related genes and each EP expression, respectively.
Fig. 3 is a staining image of cell mass of MM2 chondrocyte strain accompanying cartilage matrix. (I) is a cellar image after 2 days of culturing and (II) is that after 21 days of culturing, (III) is a cartilage matrix forming hematoxyline-eosin staining image by three-dimensional culturing of the same cell, and (IV) is an alcian blue staining image of the same.
Fig. 4 is a graph showing action of each EP agonist upon intracellular cAMP concentration in MM2 chondrocyte strain. Fig. 4 (a) shows the action of each EP agonist, wherein P<0.05 shows statistical significant difference, and (b) shows concentration-dependent action of EP2 agonist.
Fig. 5 shows a result of RT-PCR on the gene expression changes by EP2 or EP3 agonist in MM2 chondrocyte strain. Fig. 5 (a) is a group of expression acceleration genes, and (b) is a group of expression inhibition genes.
Fig. 6 shows a result of RT-PCR on the gene expression changes by EP2 or EP3 agonist in human articular cartilage primary-cultured cell. Fig. 6 (a) is a group of expression acceleration genes, and (b) is a group of expression inhibition genes.
Fig. 7 is a graph showing growth stimulating effect of each EP agonist upon human articular cartilage primary-cultured cell.
Fig. 8 is a graph showing articular cartilage repairing ability of EP2 agonist in a rat femoral condyle articular cartilage damage organ culture system.
Fig. 9 shows action of EP2 agonist upon rat femoral condyle articular cartilage damage. Fig. 9 (a) and (c) are images just after the damage, and (b) and (d) are those after 21 days of the damage, (a) and (b) are hematoxyline-eosin staining images, and (b) and (d) are alcian blue staining images.
Fig. 10 is a PCNA staining image after 7 days of organ culture of rat femur head articular cartilage. Fig. 10 (a) is an image of the control, (b) is that of EP2 agonist (1 µM) treatment, and (c) is that of EP3 agonist (1 µM) treatment.
Fig. 11 shows an evaluation method of EP agonists for cartilage regeneration ability using a rat femur cartilage damage model.

### BEST MODE FOR CARRYING OUT THE INVENTION

The present invention is explained below in detail based on Examples and Formulation Examples, but the present invention is not limited thereto. Also, in the following example, in order to evaluate the compound of the present invention, assaying accuracy and/or assaying sensitivity was improved as described below.

### Example 1:

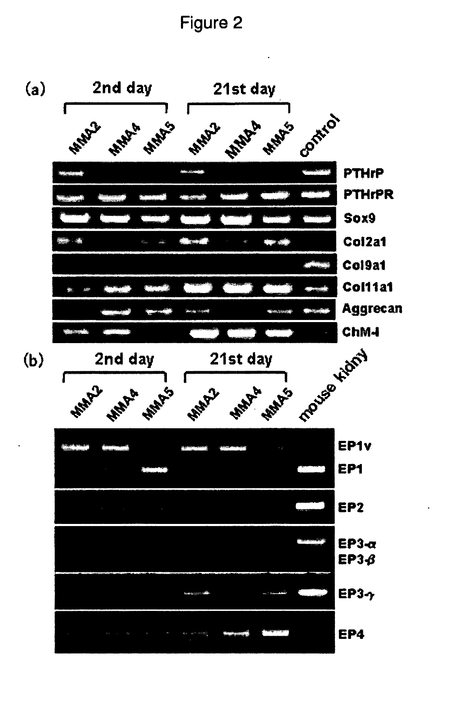

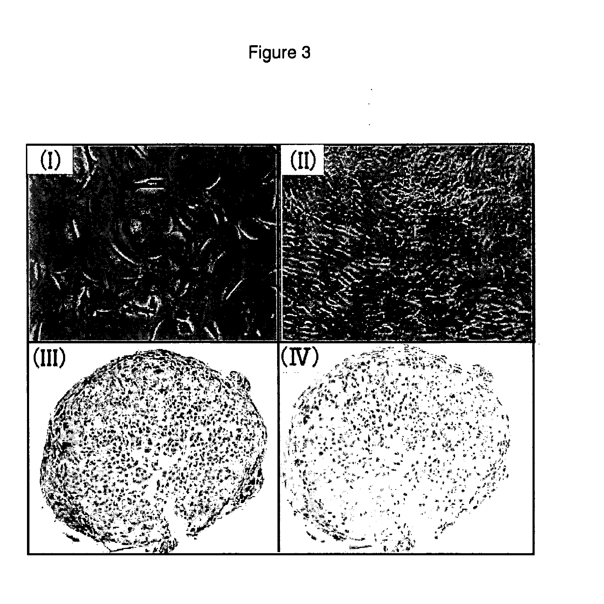

Cartilage tissues of a femur and the shinbone collected from a p53 defective mouse of 4 weeks of age (Tukada, T., Oncogene, 1992, vol. 8, pp. 3313 - 3322) were cut into pieces, treated with 0.1% collagenase and then cultured (culture conditions; 5% CO₂, 37°C, under humidification, hereinafter, this was carried out under the same conditions) using DMEM/Ham's F12 (1:1) medium (contains 10% fetal bovine serum (FBS) and antibiotics (to be referred to as DMEM/Ham's F12 medium hereinafter)). By carrying out dilution subculturing from the cell group under a 80 to 90% confluent state, a chondrocyte strain MMA2 recognized by an international deposition number FERM BP-10029 was isolated. Expression analysis of the chondrocyte strain by RT-PCR method revealed that it expressed articular cartilage -related genes of type II collagen, aggrecan and the like (Fig. 2 (a)). Also, this was a cell having characters as an articular chondrocyte, such as formation of no calcified node even after a long-term culturing and formation of a cell mass accompanied by cartilage matrix (Fig. 3 (III), (IV)). In addition, regarding the isoforms of EP2 and EP3, particularly EP3, expression of γ form was confirmed (Fig. 2 (b)).

Human primary-cultured chondrocytes were isolated from knee articular cartilages of three patients who underwent above-knee amputation due to femur osteosarcoma. In addition, other human primary-cultured chondrocytes were obtained from rheumatic arthritis patients who underwent artificial hip joint replacement. Isolation operation of these cells was also carried out by the method described in the above.

### Example 2:

Each of the chondrocytes was cultured at a cell density of 3 x 10⁵ cells/100 mm culture dish (contains 5 µM indometacin). By respectively adding (17S)-2,5-ethano-6-oxo-17,20-dimethyl-PGE₁ as a selective EP1 agonist, (5Z,9β,11α,13E)-17,17-prpano-11,16-dihydroxy-9-chloroproster-5,13,19-trienoic acid as a selective EP2 agonist, 11α,15α-dimethoxy-9-oxoproster-5Z,13E-dienoic acid as a selective EP3 agonist, and 11α,15α-dihydroxy-9-oxo-16-(3-methoxymethylphenyl)-17,18,19,20-tetranol-3,7-dithiaprost-13E-enoic acid (obtained from ONO PHARMACEUTICAL CO., LTD.) as a selective EP4 agonist, respective actions after 72 hours were evaluated.

### Example 3:

Monolayer culturing of each of the chondrocytes was started using DMEM/Ham's E12 medium containing 50 mg/ml of ascorbic acid. Medium exchange was not carried out for the first 6 days after commencement of the culturing, but carried out thereafter on every other day, and cultured cell pellet recovered on the 21st day was fixed with 20% formaldehyde and embedded in paraffin. Its section of 6 µm in thickness was stained with hematoxylin-eosin (0.1 N) hydrochloric acid solution or 0.1 % alcian blue (0.1 N) hydrochloric acid solution and photographed using an optical microscope.

### Example 4:

A low temperature section of 4 µm in thickness was prepared by incising the tibia of a newborn mouse and embedding the same in OCT (optimum cutting temperature) compound (mfd. by Sakura Seiki). This section was fixed with 4% paraformaldehyde phosphate buffer, air-dried and then digested with 20 µg/ml of proteinase K (mfd. by Dako Cytomation). The thus prepared slide glass was soaked in a hybridization buffer containing 1 µ g/ml of a 5'-FITC-labeled oligonucleotide (mfd. by Dako Cytomation) and incubated at 37°C for 6 hours in a moist chamber. Sequence of the labeled oligonucleotide probe used herein is shown in the following.
EP1 antisence;
5'-ACAGTACCCTGGCACCTGGTGTTTTATTAGCCTTGG-3' (sequence number 1)
EP2 antisence;
5'-AAAGATTGTGAAAGGCAAGGAGCATATGGCGAAGGT-3' (sequence number 2)
EP3 antisence;
5'-CAGCAGATAAACCCAGGGATCCAAGATCTGGTTCAG-3' (sequence number 3)
EP4 antisence;
5'-GGAGGAGTCTGAGGTCTCGGAAATTCGCAAAGTTCT-3' (sequence number 4)

After the hybridization, this was washed with Stringent Wash Solution (mfd. by Dako Cytomation) and subsequently allowed to react with rabbit anti-FITC/HRP antibody (mfd. by Dako Cytomation) for 1 hour. This was further allowed to react with Streptoavidin/HRP (mfd. by Dako Cytomation), and then the signal was detected using the substrate DAB chromogen solution (mfd. by Dako Cytomation).

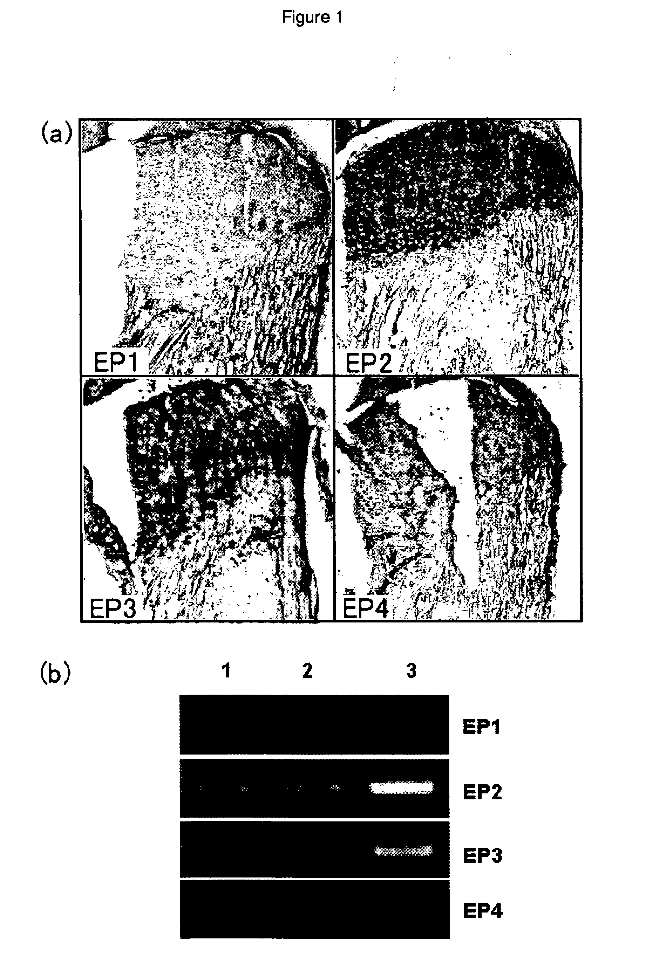

As a result, it was revealed that EP2 and EP3 are expressed in epiphysial cartilages including the articular cartilage of the tibia of newborn mouse (Fig. 1 (a)).

### Example 5:

Culturing of each chondrocyte was started at a cell density of 3 x 10⁵ cells/100 mm culture dish, and 2 days and 21 days thereafter, respective total RNA samples were prepared using Trizol Reagent (mfd. by Life Technologies) in accordance with the instructions attached thereto. The RT reaction was carried out in accordance with the instructions attached to RT-PCR kit (mfd. by Life Technologies) using 1 µg of the thus prepared total RNA. The PCR reaction was carried out in 25 µl of a reaction liquid (20 pmol sense primer, 20 pmol antisense primer, 25 mM MgCl₂, 0.2 mM dNTP, 1 unit γ Taq polymerase (mfd. by TOYOBO., LTD.)) containing 1 µl of the RT reaction liquid. The PCR reaction (in the reaction, starting reaction was carried out at 94°C for 5 minutes, and then the following reaction (denaturation reaction at 94°C for 1 minute, annealing reaction at 72°C for 1 minute and elongation reaction at 72°C for 7 minutes) was carried out 35 times) was carried out using Gene Amp 9700 (mfd. by PE Applied Biosystem). Sequences of the primers used in the above RT-PCR are shown in the following.
EP1 primer;
5'-ACCTGGTGTTTTATTAGCCTT-3' (sequence number 5)
5'-GGCCGCTGCAGGGAGTTAGAG-3' (sequence number 6)
EP2 primer;
5'-CGTGTACCTATTTCGCTTTC-3' (sequence number 7)
5'-GAGGTCCCACTTTTCCTTTA-3' (sequence number 8)
EP4 primer;
5'-CATCGACTGGACCACCAACGT-3' (sequence number 9)
5'-TCTCCTTTAACTCCCGGGCGA-3' (sequence number 10)
EP3α and EP3β primer;
5'-CCTGGGTTTATCTGCTGCTAAG-3' (sequence number 11)
5'-CTCGGTGTGTTTAATGGCAAGG-3' (sequence number 12)
EP3γ primer;
5'-CCTGGGTTTATCTGCTGCTAAG-3' (sequence number 13)
5'-CTCTGGCAAAGACTCAAAATGC-3' (sequence number 14)
β-actin primer;
5'-AAGAGAGGTATCCTGACCCT-3' (sequence number 15)
5'-TACATGGCTGGGGTGTTGAA-3' (sequence number 16)

Subsequently, the PCR products were separated by an agarose gel electrophoresis and detected by ethidium bromide staining.

As a result, expression of EP2 and EP3 was confirmed in the human cartilage tissue and primary-cultured chondrocyte (Fig. 1 (b)).

### Example 6:

Culturing of each chondrocyte was started at a cell density of 1 x 10⁴ cells/24 well culture dish, and 2 hours thereafter, each of the selective EP agonists shown in Example 2 was added thereto to continue the culturing for additional 12 hours. The intracellular cAMP concentration was measured using a cAMP assay kit (Cayman Chemical Company) and in accordance with the instructions attached to the kit, using, as a sample, lysed supernatant of the cell prepared by lysing the same with a cell lysis liquid (0.1 mM Tris/HCl buffer, pH 7.2).

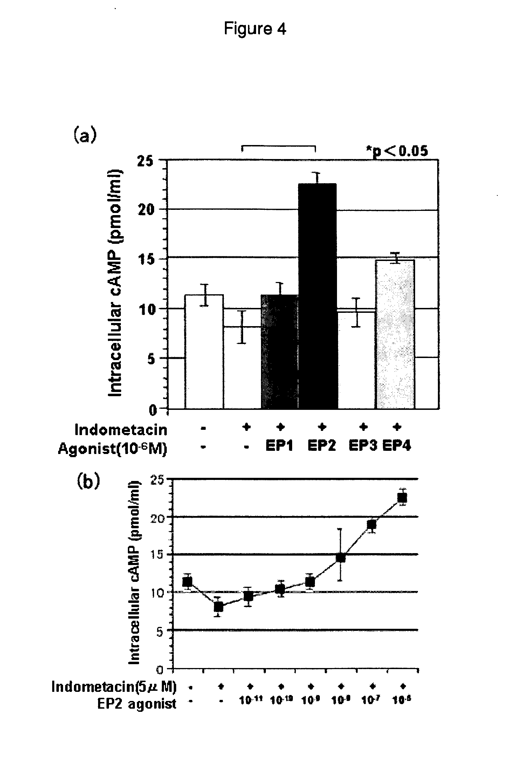

As a result of measuring intracellular cAMP concentration by adding each of the EP agonists described in Example 2 to the MMA2 chondrocyte strain, it was revealed that intracellular cAMP is increased concentration-dependently by the EP2 agonist (Fig. 4 (a), (b)).

### Example 7:

Gene expression profile of chondrocytes was analyzed by a custom-made cDNA microarray system. This system was prepared by spotting 78 species of bone- and cartilage-related mouse genes and 900 species of mouse genes (InteliGene CHIP ver.1) (mfd. by Takara Bio INC.) on a glass slide.

A chondrocyte was cultured for 72 hours in DMEM/F12 medium (contains 5 µM indometacin) in the presence or absence of each of the selective EP agonists shown in Example 2 (1 µM), and respective total RNA samples were extracted. A fluorescent cDNA probe was synthesized using 20 µg of each of the total RNA samples as the template, and using 400 U of M-MLV reverse transcriptase and Cy3 or Cy5-dUTP (Amersham Biosciences).

Each cDNA probe dissolved in a reaction buffer (6 x SSC/0.2% SDS, 5 x Denhardt's solution, 0.1 mg/ml denatured salmon sperm DNA) was allowed to hybridize with the spots on the glass slide at 65°C overnight. The slide was washed with a washing liquid (2 x SSC/0.2% SDS) twice at 55°C for 5 minutes and then once at 65°C for 5 minutes, and finally washed with 0.05 x SSC solution at room temperature for 1 minute. The hybridization signal was visualized by Affymetrix 418 Array Scanner (mfd. by Affymetrix), and analyzed by ImaGene software (mfd. by BioDiscovery). Regarding the expressed genes in which changes were confirmed, reconfirmation was carried out by the RT-PCR method.

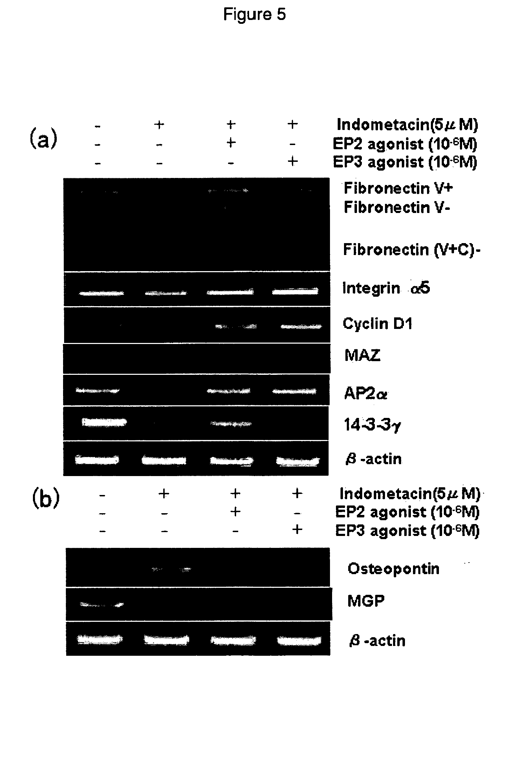

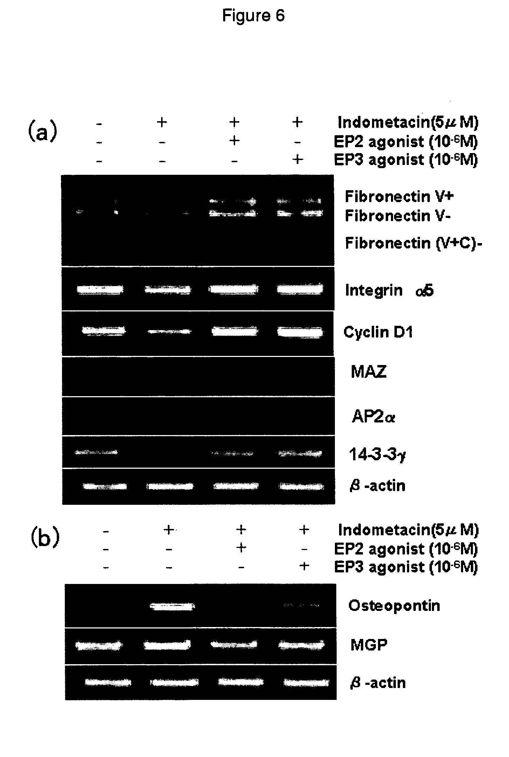

As a result of the analysis, fibronectin, integrin, cyclin D1, MAZ, AP2α and 14-3-3γ were confirmed as genes whose expression in the chondrocyte strain is stimulated by the addition of EP2 agonist or EP3 agonist (Fig. 5 (a)). On the other hand, osteopontin and MGP were confirmed as genes whose expression is reduced (Fig. 5 (b)). In addition, similar results were obtained also in the human articular cartilage primary-cultured cell (Fig. 6 (a), (b)).

### Example 8:

The cell growth activity was measured by a BrdU incorporation assay using BrU labeling, detection kit (mfd. by Boehringer Mannheim GmbH).

Culturing of each chondrocyte was started at a cell density of 2 x 10³ cells/96 well culture dish, and after adhesion of the cells, the selective EP agonist described in Example 2 (1 µM) was added thereto to start the culturing at 37°C overnight. Subsequently, the culturing was continued for 8 hours together with BrdU (final concentration 110 µM), and the labeled nucleus was detected by the method of the instructions attached to the kit.

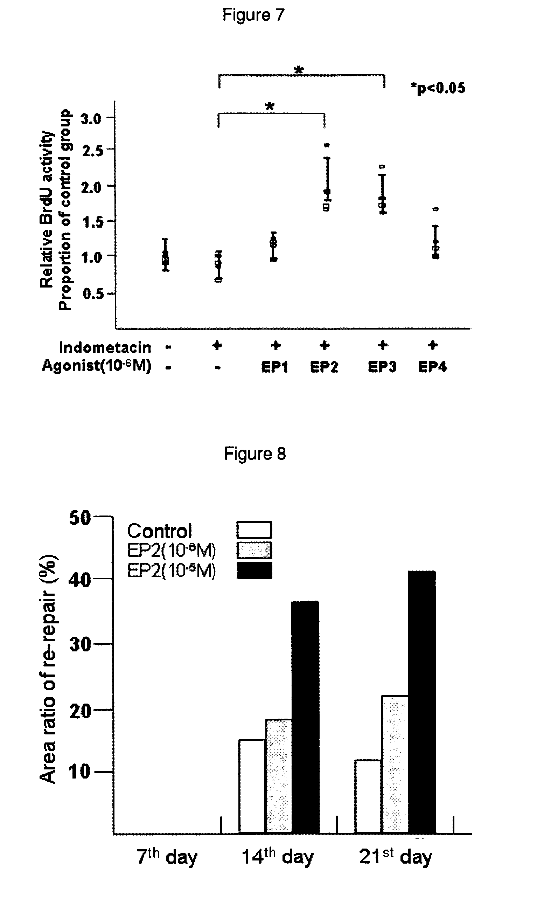

As a result of the analysis, it was revealed that the selective EP2 agonist described in Example 2 has a DNA synthesis accelerating effect for the human primary-cultured chondrocyte (Fig. 7; in the drawing, open circle shows normal cartilage, and open square shows RA articular cartilage derived cell).

### Example 9:

The cartilage deficiency model was prepared from a femoral condyle articular cartilage collected from a rat of 5 weeks of age, by cutting the cartilage layer alone such that damage is not given to the subchondral bone. The femur was soaked in the DMEM/Ham's F12 medium (contains 5 µM indometacin) and cultured for 21 days in the presence (treated group) or absence (control group) of the selective EP agonist described in Example 2 (10 µM or 1 µM). Area of the newly formed cartilage tissue was measured using Image-Pro Plus software (mfd. by Planetron) and calculated as an area ratio with the untreated lateral joint face.

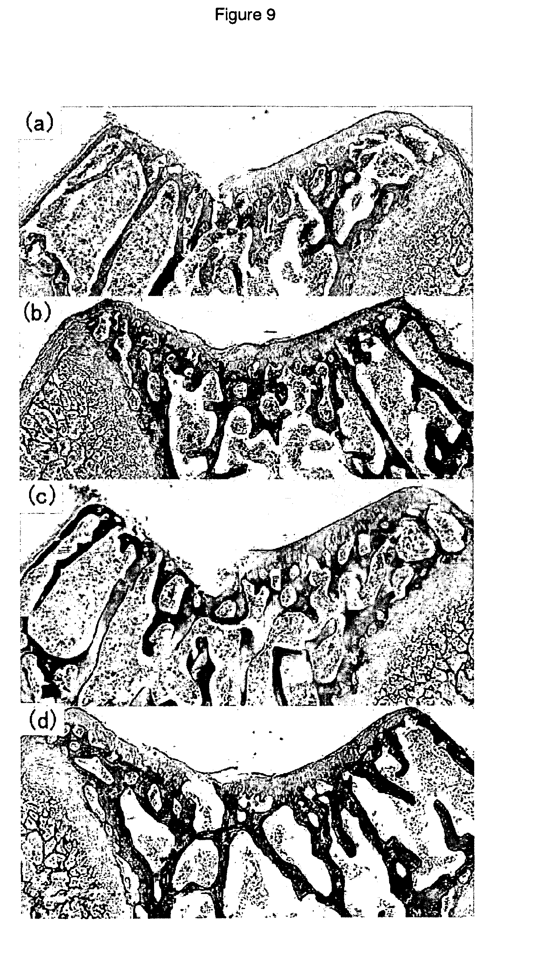

While tissue regeneration image was not observed in the control group (Fig. 9 (a)), a regeneration image having a staining affinity of similar to that of a remaining existing cartilage (right side) was observed in the treated group, and ratio of the regenerated tissue was increased periodically and concentration-dependently (Fig. 8; in the drawing, the reverse graph shows a result of untreated domain, the gray graph shows that of EP2 agonist (1 µM) treated group, and the black graph shows that of EP2 agonist (10 µM) treated group).

### Example 10:

The formalin-fixed sections were prepared on the 0th day, 7th day, 14th day and 21 st day starting from the commencement of tissue culturing described in Example 8. Each section was decalcified with EDTA (10% w/v) for 7 days and then sliced into a section of 4 µm in thickness. The hematoxylin-eosin staining and alcian blue staining were carried out by the aforementioned method.

Regarding the PCNA immuno histological staining, the prepared slide was treated with 3% hydrogen peroxide and then subjected to a blocking treatment using a blocking solution (mfd. by Dako Cytomation). Subsequently, this was incubated at 4°C overnight together with anti-PCNA antibody (final concentration; 5 µg/ml), and further allowed to undergo the reaction at room temperature for 1 hour by adding rabbit ENVISION Polymer Reagent (mfd. by Dako Cytomation) thereto. After washing, the reaction with a substrate, 3,3'-diaminobenzidine tetrahydrochloride (mfd. by Dako Cytomation), was detected. This section was contrast-stained with hematoxylin and absolute alcohol.

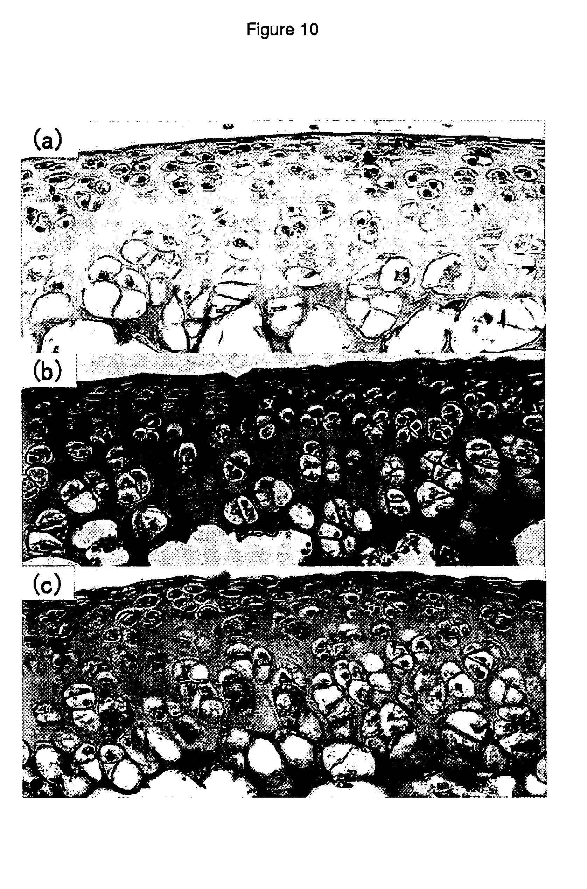

As a result of the analysis, the staining affinity was distinctively increased in the treated group of the selective EP2 agonist or EP3 agonist described in Example 2, and the effect in the EP2 agonist treated group was particularly significant (Fig. 10 (b)).

### Example 11:

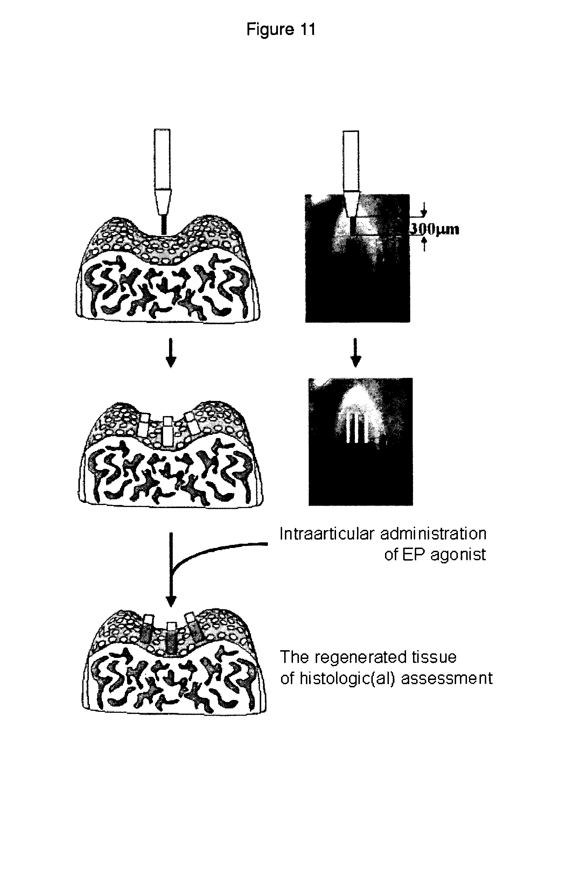

Under anesthesia, a knee joint of a rat of 6 weeks of age was incised, and a chondral defect of 300 µm in depth was prepared on the patella joint face of the femoral articular cartilage. Polymer beads which had been impregnated with the EP2 agonist or EP3 agonist were indwelled in the damaged part, and the joint was closed (Fig. 11). Thereafter, both joints were histologically evaluated after 1, 2, 4 and 8 weeks. By this evaluation, effect of the EP2 agonist or EP3 agonist on the cartilage regeneration ability can be evaluated based on the determination of safranin O-positive region, determination of type II collagen, aggrecan and the like cartilage matrixes, and PCNA staining and TUNNEL staining.

### Formulation Example 1:

The following components were admixed in a conventional manner, punched out to give 10,000 tablets each containing 0.5 mg of active ingredient.

| | | |
|---|---|---|
| • (5Z,9β,11α,13E)-17,17-propano-11,16-dihydroxy-9-chloro-20-norprosta-5,13-dienoic acid | .......5g | |
| • carcium carboxymethyl cellulose | | .......20g |
| • magnesium stearate | | .......10g |
| • microcrystalline cellulose | | .......920g |

### Formulation Example 2:

Each of the following components was mixed by a standard method and filtered through a dustproofing filter, and then 1 ml aliquots were charged into vials, which were autoclaved to thereby obtain 10,000 vials each containing 0.2 mg of the active ingredient.

| | | |
|---|---|---|
| • (5Z,9β,11α,13E)-17,17-propano-11,16-dihydroxy-9-chloro-20-norprosta-5,13-dienoic acid | .......2g | |
| • mannitol | | .......500g |
| • distilled water | | .......10L |

### Formulation Example 3:

The following components were admixed in a conventional manner, punched out to give 10,000 tablets each containing 0.5 mg of active ingredient.

| | |
|---|---|
| • 11α,15α-dimethoxy-9-oxoprosta-5Z,13E-dienoic acid | .......5g |
| • carcium carboxymethyl cellulose | .......20g |
| • magnesium stearate | .......10g |
| • microcrystalline cellulose | .......920g |

### Formulation Example 4:

Each of the following components was mixed by a standard method and filtered through a dustproofing filter, and then 1 ml aliquots were charged into vials, which were autoclaved to thereby obtain 10,000 vials each containing 0.2 mg of the active ingredient.

| | |
|---|---|
| • 11α, 15α-dimethoxy-9-oxoprosta-5Z, 13E-dienoic acid | .......2g |
| • mannitol | .......500g |
| • distilled water | .......10L |

### INDUSTRIAL APPLICABILITY

The remedy of the present invention has superior effects of stimulating chondrogenesis, stimulating chondrocyte growth, stimulating chondrocyte differentiation, inhibiting cartilage calcification and/or inhibiting cartilage degradation, and therefore, is useful in prevent and/or treatment for various bone diseases caused by cartilage disorders, or production of cartilage grafts.

It can be expected that the remedy prevents and/or treats rheumatoid arthritis, osteoporosis, osteoarthritis, osteochondral defect, cartilage damage, articular disk damage, meniscus injury, chondrodysplasia, incomplete repair and healing of bone fracture, refracture, achondroplasia, achondrogenesis, bone deformation or spondylosis deformans, dyschondrogenesis, chondrodystrophia, articular chondrocalcinosis, acute purulent arthritis, tuberculous arthritis, syphilitic arthritis, systemic lupus erythematosus, spondylosis deformans, disk herniation, injury by sports, keypuncher's disease, osteosarcoma, myeloma, osteomalacia, rickets, osteitis fibrosa, renal ostaodystrophy or bone Behcet disease, or improves functional disorders accompanied by diseases.

Futher Embodiments
1. An agent for treating cartilage-related disease comprising as an active ingredient a substance having an EP2 and/or EP3 agonist activity.
2. The agent for treating cartilage-related disease according to embodiment 1, which is an agent for treating cartilage disorder.
3. The agent for treating cartilage-related disease according to embodiment 1, which is an agent for producing a cartilage graft.
4. The agent for treating cartilage-related disease according to embodiment 2 or 3, which has one or more effects selected from stimulating chondrogenesis, stimulating chondrocyte growth, stimulating chondrocyte differentiation, inhibiting cartilage calcification and inhibiting cartilage degradation.
5. The agent for treating cartilage-related disease according to embodiment 3, which is an agent for chondrocyte culture.
6. The agent for treating cartilage-related disease according to embodiment 2 or 3, which has one or more effects selected from stimulating integrin mRNA expression, stimulating fibronectin mRNA expression, stimulating cyclin D1 mRNA expression and inhibiting osteopontin mRNA expression.
7. The agent for treating cartilage-related disease according to embodiment 4
   wherein the one or more effects selected from stimulating chondrogenesis, stimulating chondrocyte growth, stimulating chondrocyte differentiation, inhibiting cartilage calcification and inhibiting cartilage degradation is/are based on one or more effects selected from stimulating integrin mRNA expression, stimulating fibronectin mRNA expression, stimulating cyclin D1 mRNA expression and inhibiting osteopontin mRNA expression on a chondrocyte or a cartilage tissue.
8. The agent for treating cartilage-related disease according to embodiment 7, wherein the effect of stimulating chondrocyte growth is based on stimulating cyclin D1 mRNA expression.
9. The agent for treating cartilage-related disease according to embodiment 7, wherein the effect of inhibiting cartilage calcification is based on inhibiting osteopontin mRNA expression.
10. An agent for treating cartilage-related disease comprising a combination of one or more substances selected from transforming growth factor-β, insulin-like growth factor, basic fibroblast growth factor, epidermal growth factor, growth hormone and platelet-derived growth factor, and the substance having an EP2 and/or EP3 agonist activity according to embodiment 1.
11. A method for treating cartilage-related disease, which comprises administering a substance having an EP2 and/or EP3 agonist activity.
12. A method for producing a cartilage graft, which comprises adding a substance having an EP2 and/or EP3 agonist activity.
13. Use of a substance having an EP2 and/or EP3 agonist activity, for the preparation of an agent for treating cartilage disorder or for the preparation of an agent for producing a cartilage graft.
14. The agent for treating cartilage-related disease according to embodiment 1,
   wherein the substance having an EP2 agonist activity is one or more compounds selected from a compound described in EP860430, a compound described in WO99/33794, a compound described in EP974580, a compound described in W02003/74483, a compound described in WO95/19964, a compound described in WO98/28264, a compound described in WO99/19300,
   a compound described in EP0911321, a compound described in US4,132,738 and a compound described in US3,965,143.
15. The agent for treating cartilage-related disease according to embodiment 14,
   wherein the compound is one or more compounds selected from
   (1) (5Z,9β,11α,13E)-17,17-propano-11,16-dihydroxy-9-chloro-20-norprosta-5,13-dienoic acid,
   (2) (5Z,9β,11α,13E)-17,17-propano-11,16-dihydroxy-9-chloroprosta-5,13,19-trienoic acid,
   (3) trans-2-(4-(1-hydroxyhexyl)phenyl)-5-oxocyclopentaneheptanoic acid,
   (4) 2-[3-(4-tert-butylbenzyl)-N-(pyridin-3-ylsulfonyl)aminomethyl]phenoxy]acetic acid,
   (5) [1R[1α,2β(1E,4R*),3α]]-3-hydroxy-2-[4-hydroxy-4-(1-propylcyclobutyl)-1-butenyl]-5-oxocyclopentane-heptanoic acid methyl ester,
   (6) (2R,3R,4R)-4-hydroxy-2-(7-hydroxyheptyl)-3-[(E)-(4RS)-(4-hydroxy-4-methyl-1-octenyl)]cyclopentanone, and
   (7) (+1-)-15-deoxy-16-α,β-hydroxy-16-methyl PGE1 methylester.
16. The agent for treating cartilage-related disease according to embodiment 1, wherein the substance having an EP3 agonist activity is one or more compounds selected from a compound described in WO98/34916, a compound described in JP-A-8-239356, a compound described in US4,692,464, a compound described in JP-A-61-249951, a compound described in US4,863,961and a compound described in US3,985,791.
17. The agent for treating cartilage-related disease according to embodiment 16, wherein the compound is one or more compounds selected from
   (1) 11α, 15α-dimethoxy-9-oxoprosta-5Z, 13E-dienoic acid,
   (2) 2-[5-[2-[N-(diphenylmethyl)carbamoyl]ethyl]naphthalen-1-yloxy]acetic acid,
   (3) (1S,5S,6R,7R)-5-[7-hydroxy-6-[3(S)-hydroxy-3-methy)-1(E)-octenyl]bicyclo[3.3.0]oct-2-ene-3-yl]pentanoic acid,
   (4) (-)-[1(R)-[1α(Z),2β(R*),3α]]-7-[3-hydroxy-2-(2-hydroxy-3-phenxypropoxy)-5-oxocyclopentyl]-4-heptenoic acid 4-(benzoylamino)phenylester,
   (5) methyl-7-(2β-(6-(1-cyclopentyl-yl)-4R-hydroxy-4-methyl-1E,5E-hexadienyl)-3α-hydroxy-5-oxo-1R,1α-cyclopentyl)-4Z-heptenoic acid, and
   (6) 9-oxo-11α, 15α-dihydroxy-16-phenoxy-17,18,19,20-tetranorprosta-4,5,13-trans-trienoic acid methyl ester.
18. The agent for treating cartilage-related disease according to embodiment 1, wherein the compound having an EP3 agonist activity is 16-phenoxy-ω-17,18,19,20-tetranor-PGE2 methylsulfonamide or a salt thereof.
19. A method for screening the agent for treating cartilage-related disease according to embodiment 1.

## Claims

1. An agent for use in a method for treating cartilage disorder comprising as an active ingredient one or more compounds selected from
(1) (5Z,9β,11α,13E)-17,17-propano-11,16-dihydroxy-9-chloro-20-norprosta-5,13-dienoic acid,
(2) (5Z,9β,11a,13E)-17,17-propano-11,16-dihydroxy-9-chloroprosta-5,13,19-trienoic acid, and
(3) 11α,15α-dimethoxy-9-oxoprosta-5Z,13E-dienoic acid.

2. The agent according to claim 1, wherein the cartilage disorder is cartilage disorder accompanied by rheumatoid arthritis, osteoporosis, osteoarthritis, osteochondral defect, cartilage damage, articular disk damage, meniscus injury, chondrodysplasia, incomplete repair and healing of bone fracture, refracture, achondroplasia, achondrogenesis, bone deformation or spondylosis deformans, dyschondrogenesis, chondrodystrophia, articular chondrocalcinosis, acute purulent arthritis, tuberculous arthritis, syphilitic arthritis, systemic lupus erythematosus, spondylosis deformans, disk herniation, injury by sports, keypuncher's disease, osteosarcoma, myeloma, osteomalacia, rickets, osteitis fibrosa, renal ostaodystrophy or bone Behcet disease.

3. The agent according to claim 1, wherein the cartilage disorder is cartilage disorder accompanied by osteochondral defect, cartilage damage, articular disk damage, meniscus injury, chondrodysplasia, incomplete repair and healing of bone fracture, refracture, achondroplasia, achondrogenesis, bone deformation or spondylosis deformans, dyschondrogenesis, chondrodystrophia, articular chondrocalcinosis, injury by sports or keypuncher's disease.

4. The agent according to claim 1, which has one or more effects selected from stimulating chondrogenesis, stimulating chondrocyte growth, stimulating chondrocyte differentiation, inhibiting cartilage calcification and inhibiting cartilage degradation.

5. The agent according to claim 1, which has one or more effects selected from stimulating integrin mRNA expression, stimulating fibronectin mRNA expression, stimulating cyclin D1 mRNA expression and inhibiting osteopontin mRNA expression.

6. The agent according to claim 4, wherein the one or more effects selected from stimulating chondrogenesis, stimulating chondrocyte growth, stimulating chondrocyte differentiation, inhibiting cartilage calcification and inhibiting cartilage degradation is/are based on one or more effects selected from stimulating integrin mRNA expression, stimulating fibronectin mRNA expression, stimulating cyclin D1 mRNA expression and inhibiting osteopontin mRNA expression on a chondrocyte or a cartilage tissue.

7. The agent according to claim 6, wherein the effect of stimulating chondrocyte growth is based on stimulating cyclin D1 mRNA expression.

8. The agent according to claim 6, wherein the effect of inhibiting cartilage calcification is based on inhibiting osteopontin mRNA expression.

9. An agent for use in a method for stimulating chondrogenesis comprising as an active ingredient one or more compounds selected from
(1) (5Z,9β,11α,13E)-17,17-propano-11,16-dihydroxy-9-chloro-20-norprosta-5,13-dienoic acid,
(2) (5Z,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid.

10. An agent for use in a method for stimulating chondrocyte growth comprising as an active ingredient one or more compounds selected from
(1)
(5Z,9β,11α, 13E)-17,17-propano-11,16-dihydroxy-9-chloro-20-norprosta-5,13-dienoic acid,
(2) (5Z,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid.

11. An agent for use in a method for stimulating chondrocyte differentiation comprising as an active ingredient one or more compounds selected from
(1) (5Z,9β,11α,13E)-17,17-propano-11,16-dihydroxy-9-chloro-20-norprosta-5,13-dienoic acid,
(2) (SZ,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid.

12. An agent for use in a method for inhibiting cartilage calcification comprising as an active ingredient one or more compounds selected from
(1)
(5Z,9β,11α,13E)-17,17-propano-11,16-dihydroxy-9-chloro-20-norprosta-5,13-dienoic acid,
(2) (5Z,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid.

13. An agent for use in a method for inhibiting cartilage degradation comprising as an active ingredient one or more compounds selected from
(1)
(5Z,9β,11α,13E)-17,17-propano-11,16-dihydroxy-9-chloro-20-norprosta-5,13-dienoic acid,
(2) (5Z,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid.

14. An agent for use in a method for treating cartilage disorder accompanied by osteochondral defect, cartilage damage, articular disk damage, meniscus injury, chondrodysplasia, incomplete repair and healing of bone fracture, refracture, achondroplasia, achondrogenesis, bone deformation or spondylosis deformans, dyschondrogenesis, chondrodystrophia, articular chondrocalcinosis, injury by sports or keypuncher's disease comprising as an active ingredient one or more compounds selected from
(1) (5Z,9β,11α,13E)-17,17-propano-11,16-dihydroxy-9-chloro-20-norprosta-5,13-dienoic acid,
(2) (5Z,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid, based on an activity of stimulating chondrogenesis of the compounds.

15. Use of an agent for manufacturing a medicament for treating cartilage disorder, wherein the agent comprises as an active ingredient one or more compounds selected from
(1) (5Z,9β,11α,13E)-17,17-propano-11,16-dihydroxy-9-chloro-20-norprosta-5,13-dienoic acid,
(2) (5Z,9β,11α,13E)-17,17-propano-11,16-dihydroxy-9-chloroprosta-5,13,19-trienoic acid, and
(3) 11α,15α-dimethoxy-9-oxoprosta-5Z,13E-dienoic acid.


SEQUENCE LISTING

<110>  ONO PHARMACEUTICAL CO.,LTD.

<120>  Remedy for Cartilage-Related Diseases

<130>  EP75170IHVSZpau

<140>  not yet assigned
<141>  2004-07-23

<150>  04 748 097.5
<151>  2004-07-23

<150>  PCT/JP2004/010890
<151>  2004-07-23

<150>  JP 2003-280191
<151>  2003-07-25

<160>  16

<170>  PatentIn version 3.1

<210>  1
<211>  36
<212>  DNA
<213>  Artificial

<220>
<223>  Antisence oligonucleotide

<400>  1
acagtaccct ggcacctggt gttttattag ccttgg                                36

<210>  2
<211>  36
<212>  DNA
<213>  Artificial

<220>
<223>  Antisence oligonucleotide

<400>  2
aaagattgtg aaaggcaagg agcatatggc gaaggt                                36

<210>  3
<211>  36
<212>  DNA
<213>  Artificial

<220>
<223>  Antisence oligonucleotide

<400>  3
cagcagataa acccagggat ccaagatctg gttcag                                36

<210>  4
<211>  36
<212>  DNA
<213>  Artificial

<220>
<223>  Antisence oligonucleotide

<400>  4
ggaggagtct gaggtctcgg aaattcgcaa agttct                                36

<210>  5
<211>  21
<212>  DNA
<213>  Artificial

<220>
<223>  PCR primer oligonucleotide

<400>  5
acctggtgtt ttattagcct t                                                21

<210>  6
<211>  21
<212>  DNA
<213>  Artificial

<220>
<223>  PCR primer oligonucleotide

<400>  6
ggccgctgca gggagttaga g                                                21

<210>  7
<211>  20
<212>  DNA
<213>  Artificial

<220>
<223>  PCR primer oligonucleotide

<400>  7
cgtgtaccta tttcgctttc                                                  20

<210>  8
<211>  20
<212>  DNA
<213>  Artificial

<220>
<223>  PCR primer oligonucleotide

<400>  8
gaggtcccac ttttccttta                                                  20

<210>  9
<211>  21
<212>  DNA
<213>  Artificial

<220>
<223>  PCR primer oligonucleotide

<400>  9
catcgactgg accaccaacg t                                                21

<210>  10
<211>  21
<212>  DNA
<213>  Artificial

<220>
<223>  PCR primer oligonucleotide

<400>  10
tctcctttaa ctcccgggcg a                                                21

<210>  11
<211>  22
<212>  DNA
<213>  Artificial

<220>
<223>  PCR primer oligonucleotide

<400>  11
cctgggttta tctgctgcta ag                                               22

<210>  12
<211>  22
<212>  DNA
<213>  Artificial

<220>
<223>  PCR primer oligonucleotide

<400>  12
ctcggtgtgt ttaatggcaa gg                                                22

<210>  13
<211>  22
<212>  DNA
<213>  Artificial

<220>
<223>  PCR primer oligonucleotide

<400>  13
cctgggttta tctgctgcta ag                                                22

<210>  14
<211>  22
<212>  DNA
<213>  Artificial

<220>
<223>  PCR primer oligonucleotide

<400>  14
ctctggcaaa gactcaaaat gc                                                22

<210>  15
<211>  20
<212>  DNA
<213>  Artificial

<220>
<223>  PCR primer oligonucleotide

<400>  15
aagagaggta tcctgaccct                                                   20

<210>  16
<211>  20
<212>  DNA
<213>  Artificial

<220>
<223>  PCR primer oligonucleotide

<400>  16
   tacatggctg gggtgttgaa                                                    20